Europäisches Patentamt

European Patent Office

Office européen des brevets

(11) Numéro de publication: **0 423 302 B1**

# FASCICULE DE BREVET EUROPEEN

(45) Date de publication de fascicule du brevet: **26.04.95** (51) Int. Cl.⁶: **C12N 15/15**, C12N 15/62

(21) Numéro de dépôt: **90907160.7**

(22) Date de dépôt: **27.04.90**

(86) Numéro de dépôt internationale :
**PCT/FR90/00306**

(87) Numéro de publication internationale :
**WO 90/13646 (15.11.90 90/26)**

(54) **APPLICATION DE NOUVEAUX FRAGMENTS D'ADN EN TANT OUE SEOUENCE CODANT POUR UN PEPTIDE SIGNAL POUR LA SECRETION DE PROTEINES MATURES PAR DES LEVURES RECOMBINANTES, CASSETTES D'EXPRESSION, LEVURES TRANSFORMEES ET PROCEDE DE PREPARATION DE PROTEINES CORRESPONDANT.**

(30) Priorité: **28.04.89 FR 8905687**

(43) Date de publication de la demande:
**24.04.91 Bulletin 91/17**

(45) Mention de la délivrance du brevet:
**26.04.95 Bulletin 95/17**

(84) Etats contractants désignés:
**AT BE CH DE DK ES FR GB IT LI LU NL SE**

(56) Documents cités:
**EP-A- 0 158 564       EP-A- 0 200 655**
**EP-A- 0 225 633       EP-A- 0 252 854**
**EP-A- 0 273 800       EP-A- 0 349 451**

**The Journal of Biological Chemistry, vol. 257, no. 24, 26.12.1982, American Society of Biological Chemists, Inc., (Baltimore, MD,US), J. Kaput et alpp. 15054-15058**

(73) Titulaire: **TRANSGENE S.A.**
**11, rue de Molsheim**
**F-67000 Strasbourg (FR)**

(72) Inventeur: **ACHSTETTER, Tilman**
**Uhlandweg 11**
**D-7602 Oberkirch (DE)**
Inventeur: **NGUYEN, Martine**
**21, rue du Paradis**
**F-67670 Wittersheim (FR)**
Inventeur: **LEMOINE, Yves**
**4, rue des Alisiers**
**F-67100 Strasbourg (FR)**
Inventeur: **REICHHART, Jean-Marc**
**34, rue de Rotterdam**
**F-67000 Strasbourg (FR)**

(74) Mandataire: **Warcoin, Jacques et al**
**Cabinet Régimbeau,**
**26, avenue Kléber**
**F-75116 Paris (FR)**

Journal of Bacteriology, vol. 171, no. 11, Novembre 1989, American Society for Microbiology, (Baltimore, MD, US), F. Klebl et al. pp. 6259-6264

## Description

La présente invention a pour objet de nouveaux fragment d'ADN et leur utilisation en tant que fragments d'ADN codant pour un peptide signal utile pour la sécrétion de protéines hétérologues par des cellules eucaryotes (animales ou végétales) ou procaryotes (bactéries), plus particulièrement des levures telles que des souches de *Saccharomyces*.

Lors de la préparation d'une protéine hétérologue par les techniques de l'ADN recombinant, un des objectifs souvent poursuivis est l'obtention d'un produit qui est sécrété dans le milieu de culture des cellules qui synthétisent la proteine hétérologue. En effet, dans le cas notamment de la préparation d'une protéine d'intérêt industriel, destinée à être produites en grande quantité, il est souhaitable, afin qu'elle conserve les propriétés désirées, que la protéine soit produite sous forme mature, c'est à dire dépourvue de tout acide aminé ou séquence peptidique supplémentaire demeurée fusionnée à la protéine. Par ailleurs, il peut être intéressant qu'elle soit sécrétée dans le milieu de culture afin de faciliter les opérations de récupération et purification.

Les protéines qui sont sécrétées par une cellule, en particulier par une cellule eucaryote, sont trés généralement synthétisées sous forme d'un précurseur polypeptidique comprenant un fragment correspondant à la protéine mature (forme active) et un fragment N-terminal dit fragment "pré", aussi appelé peptide signal, qui intervient dans le mécanisme de sécrétion de la protéine par la cellule. En outre, ce précurseur polypeptidique peut comprendre un ou des fragments additionnels, appelés fragments "pro". Dans ce dernier cas, le précurseur polypeptidique est appelé précurseur "pré-pro" ou premier précurseur. Un fragment "pro" est, dans la majorité des cas, inséré entre le peptide signal et le fragment correspondant à la protéine mature, bien que ceci ne soit pas une règle absolue.

Un peptide signal initie (i) l'insertion de la protéine dans la membrane cellulaire, (ii) la translocation de la protéine au travers de la membrane cellulaire, ou (iii) l'entrée de la protéine dans le réticulum endoplasmique de la cellule en vue de la sécrétion de la protéine par la voie du réticulum endoplasmique. Une fois que le peptide signal a rempli son office, il est normalement detaché par clivage protéolytique pour libérer une protéine mature ou un deuxième précurseur appelé précurseur "pro" qui, tout comme le premier précurseur, n'a pas d'activité biologique, ou qui n'a pas l'activité complète de la protéine mature.

Un fragment "pro" est utile dans la mesure où il bloque ou modifie l'activité de la protéine, permettant ainsi de protéger la cellule contre les effets toxiques éventuels de la protéine ou de protéger la protéine contre d'eventuelles modifications ou dégradations. Il peut aussi intervenir dans une certaine mesure dans le mechanisme de sécrétion En fin du processus de sécrétion, le fragment "pro" du deuxième précurseur est détaché par clivage protéolytique pour libérer une protéine mature (forme active).

A la jonction du fragment "pré" et du fragment "pro", ainsi que à la jonction du fragment "pro" et du fragment correspondant à la protéine mature, doit se trouver un site de clivage protéolytique qui est reconnu par une des protéases de la cellule dans laquelle la protéine est synthétisée. Ce site de clivage protéolytique est généralement constitué d'une séquence de 2 ou 3 acides aminés ou plus (appelée par la suite séquence de protéolyse) qui est accessible à la protéase sur le précurseur "pro" et qui, si elle existe sur le fragment correspondant à la protéine mature, n'est pas accessible. Trois cas sont a priori possibles, illustrés ci-dessous en référence à la jonction des fragments "pré" et "pro":

- ou bien la protéase coupe en tête de séquence et, par conséquent, la séquence de protéolyse doit se lire sur le fragment "pro";
- ou bien la protéase coupe en queue de séquence et, par conséquent, la séquence de protéolyse doit se lire sur le fragment "pré";
- ou bien la protéase coupe en milieu de séquence et, par conséquent, la séquence de protéolyse doit a lire à cheval sur les fragments " pré" et "pro".

Ces considérations s'appliquent bien sur de manière similaire au site de clivage placé à la jonction du fragment "pro" et du fragment correspondant à la protéine mature.

Pour la construction de précurseurs synthétiques par les méthodes du génie génétique on peut être amené à utiliser des fragments naturels (c'est-à-dire tels que trouvés dans la nature) et à insérer en bonne place un nouveau site de clivage protéolytique ou certains acides aminés de manière à reconstituer un nouveau site de clivage protéolytique en association avec les fragments, ce nouveau site de clivage étant bien sur reconnu par une des protéases de la cellule dans laquelle le précurseur synthétique est exprimé.

Un exemple du type de synthèse et du mode de sécrétion décrit ci-dessus est illustré par le cas de la phéromone sexuelle α de la levure *S. cerevisiae*, aussi appelé facteur α (codé à partir du gène MFα1 ou MFα2). Le Facteur α est en effet synthétisé sous forme de précurseur "pré-pro" tel que décrit dans Kurjan et Herskowitz, Cell (1982) 30: 933. La séquence d'acides aminés du fragment "pré" du précurseur du Facteur α est Met Arg Phe Pro Ser Ile Phe Thr Ala Val Leu Phe Ala Ala Ser Ser Ala Leu Ala tandis que celle du fragment "pro" du précurseur du Facteur α est

Ala Pro Val Asn Thr Thr Thr Glu Asp Glu Thr Ala Gln Ile Pro Ala

Glu Ala Val Ile Gly Tyr Ser Asp Leu Glu Gly Asp Phe Asp Val Ala Val Leu Pro

Phe Ser Asn Ser Thr Asn Asn Gly Leu Leu Phe Ile Asn Thr Thr Ile Ala Ser Ile

Ala Ala Lys Glu Glu Gly Val Ser Leu Asp.

De manière surprenante, il a maintenant été trouvé que l'extrémité N-terminale du précurseur d'une enzyme de levure, cette extrémité N-terminale ayant la séquence d'acides aminés :

Arg-Phe-Ser-Thr-Thr-Leu-Ala-Thr-Ala-Ala-Thr-Ala-Leu-Phe-Phe-Thr-Ala-Ser-Gln-

Val-Ser-Ala,

peut être utilisée à titre de peptide signal pour la sécrétion de protéines hétérologues, ainsi que différents variants de cette extrémité N-terminale. Par "proteine hétérologue" on signifie une protéine qui n'est pas produite naturellement par la cellule hôte ou bien qui est codée par une séquence qui ne provient pas de la cellule hôte.

Conformément à ceci, la présente invention à pour objet un fragment d'ADN isolé qui code pour un peptide dont la séquence d'acides aminés presente un degré d'homologie d'au moins 60%, de manière préférentielle d'au moins 80%, avec la séquence d'acides aminés (I) ou (II), de préférence avec la séquence (II). Les séquences (I) et (II) sont comme suit:

(I)      Arg-Phe-Ser-Thr-Thr-Leu-Ala-Thr-Ala-Ala-Thr-Ala-Leu-Phe-Phe-Thr-

Ala-Ser-Gln.

(II)   Arg-Phe-Ser-Thr-Thr-Leu-Ala-Thr-Ala-Ala-Thr-Ala-Leu-Phe-Phe-Thr-

Ala-Ser-Gln-Val-Ser-Ala.

Par "fragment d'ADN isolé" on signifie un fragment d'ADN dont l'extrémité 3' n'est pas liée par liaison covalente à un fragment d'ADN codant pour une enzyme ayant une activité $\beta$-1,3 glucanase telle que en particulier décrite dans Klebl & Tanner, J. Bact, Nov 1989, 171: 6259.

Plus particulièrement, un fragment d'ADN selon l'invention code pour un peptide comprenant la séquence d'acides aminés (III) suivante :

(III) $R_1$-$R_2$-$R_3$-Thr-Thr-$R_4$-Ala-Thr-Ala-Ala-Thr-Ala-Leu-Phe-
        1          5               10

Phe-Thr-Ala-$R_5$-$R_6$
  15        19

dans laquelle:

$R_1$ est un acide aminé selectionné parmi Arg et Lys,

$R_2$ et $R_6$ sont chacun un acide aminé sélectionné de manière indépendante parmi Ala, Asn, Cys, Gln, Gly, His, Ile, Leu, Met, Phe, Pro, Ser, Thr, Trp, Tyr et Val,

$R_3$ et $R_5$ sont chacun un acide aminé sélectionné de manière indépendante parmi Asp, Gly, Asn, Pro et Ser, et

$R_4$ est un acide aminé sélectionné parmi Val, Leu, Ala, Cys, Phe, Ile et Met.

De manière préférée, un fragment d'ADN selon l'invention code pour un peptide comprenant la séquence d'acides aminés (IV) suivante:

(IV)      $R_1$-$R_2$-$R_3$-Thr-Thr-$R_4$-Ala-Thr-Ala-Ala-Thr-Ala-Leu-Phe-Phe-Thr-Ala-$R_5$-$R_6$-$R_7$

dans laquelle :

$R_1$ est un acide aminé selectionné parmi Arg et Lys,

$R_2$ et $R_6$ sont chacun un acide aminé sélectionné de manière indépendante parmi Ala, Asn, Cys, Gln, Gly, His, Ile, Leu, Met, Phe, Pro, Ser, Thr, Trp, Tyr et Val,

$R_3$ et $R_5$ sont chacun un acide aminé sélectionné de manière indépendante parmi Asp, Gly, Asn, Pro et Ser,

$R_4$ est un acide aminé sélectionné parmi Val, Leu, Ala, Cys, Phe, Ile et Met,

$R_7$ est une séquence de protéolyse.

$R_7$ est préférentiellement une séquence de protéolyse $R_8$-$R_9$-$R_{10}$ dans laquelle :

$R_8$ est un acide aminé sélectionné parmi Ala, Val, Ser, Cys, Gly, Ile, Leu, Thr,

$R_9$ est un acide aminé sélectionné parmi Ala, Arg, Cys, Gln, Gly, His, Ile, Leu, Met, Phe, Pro, Ser, Thr, Trp, Tyr et Val et

$R_{10}$ est un acide aminé sélectionné parmi Ala, Cys, Gly, Leu, Pro, Gln, Ser et Thr.

Selon l'invention, un fragment d'ADN préféré code pour un peptide comprenant une séquence d'acides aminés sélectionnée parmi les séquences d'acides aminés (V), (VI), (VII) et (VIII) suivantes :

(V)      Arg-Phe-Ser-Thr-Thr-Leu-Ala-Thr-Ala-Ala-Thr-Ala-Leu-Phe-Phe-Thr-Ala-Ser-Gln

(VI)     Arg-Phe-Ser-Thr-Thr-Val-Ala-Thr-Ala-Ala-Thr-Ala-Leu-Phe-Phe-Thr-Ala-Ser-Gln

(VII)    Arg-Phe-Ser-Thr-Thr-Leu-Ala-Thr-Ala-Ala-Thr-Ala-Leu-Phe-Phe-Thr-Ala-Ser-Gln-$R_7$

dans laquelle $R_7$ est tel que défini ci-dessus.

(VIII)   Arg-Phe-Ser-Thr-Thr-Val-Ala-Thr-Ala-Ala-Thr-Ala-Leu-Phe-Phe-Thr-Ala-Ser-Gln-$R_7$

dans laquelle $R_7$ est tel que défini ci-dessus.

De manière tout à fait préférée, $R_7$ est une sequence dans laquelle $R_8$ est Val, $R_9$ est Ser et $R_{10}$ est Ala.

Il est tout particulièrement préféré qu'un fragment d'ADN selon l'invention ait pour séquence nucléotidique l'une des séquences (IX), (X), (XI) et (XII) suivantes :

(IX)  CGT TTC TCT ACT ACA GTC GCT ACT GCA GCT ACT GCG CTA
       TTT TTC ACA GCC TCC CAA,

(X)   CGT TTC TCT ACT ACA GTC GCT ACT GCA GCT ACT GCG CTA
       TTT TTC ACA GCC TCC CAA,

(XI)  CGT TTC TCT ACT ACA GTC GCT ACT GCA GCT ACT GCG CTA
       TTT TTC ACA GCC TCC CAA GTT TCA GCT,

(XII) CGT TTC TCT ACT ACA CTC GCT ACT GCA GCT ACT GCG CTA
       TTT TTC ACA GCC TCC CAA GTT TCA GCT.

Les peptides codés par les fragment d'ADN selon l'invention comprennent une région hydrophobe possédant une structure en hélice α comprise entre l'acide aminé en position 3 et l'acide aminé en position 18. Cette structure contribue à les rendre aptes à une utilisation comme peptide signal. Il est connu que la partie hydrophobe des séquences signal est composée en majorité des acides aminés Ala, Cys, Phe, Ile, Leu, Met, Val. On peut donc aussi prévoir que certaines modifications d'acides aminés n'engendrent pas de modification dans l'aptitude du peptide signal à jouer son rôle.

Sous un autre aspect, l'invention propose par conséquent l'application d'un fragment d'ADN selon l'invention à titre de fragment d'ADN codant pour un peptide signal utile pour la sécrétion d'une protéine hétérologue par une cellule hôte dans laquelle la protéine hétérologue est synthétisée. D'une manière générale, l'invention peut être mise en oeuvre dans une cellule procaryote ou eucaryote, de préférence dans cette dernière. La cellule eucaryote peut être par exemple, une cellule de mammifère ou de levure. De manière tout à fait préférée, la sécrétion d'une protéine hétérologue à l'aide d'un peptide signal codé par un fragment selon l'invention est réalisée par une cellule de levure, par exemple du genre *Saccharomyces*, plus particulièrement de l'espèce *S. cerevisiae*.

Bien entendu, en tant que fragments d'ADN codant pour un peptide signal, les fragments d'ADN selon l'invention seront précédés d'un codon d'initiation de la traduction, généralement d'un codon codant pour une méthionine, en particulier un ATG.

Les fragments d'ADN selon l'invention peuvent être utilisés pour la construction d'une cassette d'expression d'une protéine, précédés du codon d'initiation, seuls ou en combinaison avec d'autres composants, par exemple un fragment "pro". Ces fragments d'ADN peuvent être préparés par synthèse chimique, au moyen d'un synthétiseur d'oligonucléotides par une technique connue de l'homme du métier.

L'invention concerne également une cassette d'expression d'une protéine hétérologue comprenant un fragment d'ADN selon l'invention à titre de fragment d'ADN codant pour le peptide signal de la dite protéine hétérologue. De manière détaillée, une cassette d'expression selon l'invention qui comporte donc l'information nécessaire à la sécrétion d'une protéine hétérologue mature comprend, de façon séquentielle, au moins:

a) un fragment d'ADN comportant des signaux d'initiation de transcription et de traduction,
b) un fragment d'ADN selon l'invention,
c) un fragment d'ADN codant pour une protéine hétérologue mature (avec codon de fin de traduction).

Dans une première variante, on peut fusionner directement le fragment b) en phase avec le fragment c), dans la mesure où, à la jonction du fragment b) et du fragment c), il existe une séquence d'ADN codant pour un site de clivage protéolytique de manière à permettre la libération d'une protéine mature en fin du processus d'expression et de sécrétion. De manière préférée, la séquence d'ADN codant pour un site de clivage protéolytique se lit sur le fragment b).

Dans une deuxième variante, une cassette d'expression selon l'invention comprend en outre un fragment d'ADN b') codant pour un fragment peptidique "pro". Ce fragment b') est fusionné en phase avec le fragment b) et le fragment c), dans la mesure où, à la jonction des fragments b) et b') d'une part et des fragments b') et c) d'autre part, il existe une séquence d'ADN codant pour un site de clivage protéolytique.

De nombreux fragments b') peuvent être utilisés dans les cassettes selon l'invention. En particulier, divers fragments b') peuvent être construits de manière synthétique. A titre d'exemple, on indique ci-dessous la construction d'un fragment b') synthétique à partir de la séquence d'ADN codant pour le fragment "pro" du précurseur du Facteur α. Cette séquence peut être utilisée en totalité ou en partie. Dans un mode de réalisation particulier, on utilise cette séquence délétée de la partie codant pour les acides

EP 0 423 302 B1

aminés en position 3 à 42 sur le fragment "pro"; c'est-à-dire la séquence codant pour :

**Ala Pro Gly Leu Leu Phe Ile Asn Thr Thr**

**Ile Ala Ser Ile Ala Ala Lys Glu Glu Gly Val Ser Leu Asp.**

Pour former le fragment b'), cette dernière séquence d'ADN est suivie d'une séquence codant pour (i) un peptide comprenant un site de clivage protéolytique ou (ii) un site de clivage protéolytique, ce dernier mode de réalisation étant préféré. Tout particulièrement, ce dernier site de clivage est Lys-Arg ou Arg-Arg, celui-ci etant reconnu par l'endopeptidase de levure yscF qui est codée par le gène KEX2 et qui coupe au niveau de l'extrémité C-terminale du dipeptide Lys-Arg ou Arg-Arg. En résumé, un fragment b') particulier code pour

**Ala Pro Gly Leu Leu Phe Ile Asn**

**Thr Thr Ile Ala Ser Ile Ala Ala Lys Glu Glu Gly Val Ser Leu Asp Lys Arg.**

La séquence a) comprend en particulier un promoteur fonctionnel dans la cellule dans laquelle on souhaite synthétiser la protéine hétérologue codée par le fragment c), de préférence un promoteur fonctionnel chez la levure. On peut citer par exemple des promoteurs constitutifs de levure dont la fonctionnalité a été confirmée par la transcription de gènes codant pour des protéines hétérologues codant pour des protéines hétérologues tels que les promoteurs PGK, ENO1, MFα1, ou encore des promoteurs inductibles, tels que PH05, GAL1. Par exemple, lorsqu'on utilise dans la cassette d'expression des éléments du gène MFα1 de levure, on pourra utiliser le promoteur du gène MFα1.

Enfin, les cassettes d'expression peuvent aussi comprendre un fragment d'ADN d) comportant des signaux de terminaison de la transcription, de préférence fonctionnels chez la levure, par exemple celui du gène PGK.

De façon générale, une cassette d'expression selon l'invention peut être introduite dans une cellule procaryote ou eucaryote, de préférence eucaryote telle que cellule de mammifère ou de levure; une cellule de levure étant tout particulièrement préférée. Cette introduction peut être realisée en plaçant la cassette dans un plasmide à replication autonome ou dans une construction destinée à l'intégration pour être introduite directement dans le génome de la levure, de manière à obtenir dans les deux cas une cellule transformée.

Lorsque le plasmide est autonome, il comportera des éléments assurant sa partiton et sa réplication; par exemple, une origine de réplication telle que celle du plasmide 2μ de levure. En outre, le plasmide pourra comporter des éléments de sélection tel que le gène URA3 ou LEU2 qui assurent la complémentation de levure ura3 ou leu2. En particulier, on pourra avantageusement utiliser le gène URA3 délété de son promoteur (URA3-d).

Ces plasmides peuvent également comporter des éléments assurant leur réplication dans les bactéries, lorsque le plasmide doit être un plasmide navette, par exemple une origine de réplication telle que celle du pBR322, un gène marqueur de sélection tel que Amp$^R$ et/ou d'autres éléments connus de l'homme du métier.

En accord avec ce qui précède, la présente invention concerne également une cellule transformée par (i) un fragment d'ADN selon l'invention ou (ii) une cassette d'expression selon l'invention, soit inserée dans un plasmide, soit intégrée dans le génome de la cellule.

Lorsque le promoteur est celui du gène MFα1, la cellule de levure transformée est de préférence de type sexuel MATα. On utilisera, par exemple, une souche de génotype ura3 ou leu2 ou autre, complémentée par le plasmide pour assurer le maintien du plasmide dans la levure par une pression de sélection appropriée.

Enfin, l'invention a pour objet un procédé de préparation d'une protéine hétérologue, caractérisé en ce que l'on cultive un cellule selon l'invention et, en ce que l'on récupère la dite protéine dans le milieu de culture. Ce procédé s'applique à la preparation de toute protéine de nature hétérologue. Parmi ces protéines, on peut notamment citer l'hirudine ou des défensines, par exemple la défensine A.

Plus particulièrement, l'invention concerne un procédé de sécrétion de l'hirudine sous forme mature à partir des souches de levures transformées selon l'invention.

7

L'invention s'applique tout particulièrement bien à la production d'hirudine, c'est pourquoi un des exemples illustratifs de l'invention concerne cette protéine. En effet l'hirudine, dont la source principale se trouve dans les glandes salivaires des sangsues médicinales est un inhibiteur très spécifique et très efficace de la thrombine. Il s'agit donc d'un agent thérapeutique très intéressant dont l'utilisation en clinique exige une très grande pureté du produit, et qui est donc un candidat intéressant à la production par génie génétique.

Un certain nombre de variants naturels de l'hirudine ont été identifiés et désignés par HV1, HV2, HV3. Par la suite ces variants naturels ainsi que d'autres analogues ont été préparés par génie génétique dans diverses cellules hôtes, comme cela est décrit par exemple dans les publications européennes de brevet EP-A-0200655, EP-A-0273800 au nom de la Demanderesse. La comparaison de l'hirudine synthétisée par *Escherichia coli* (*E. coli*) et par une levure du genre *S. cerevisiae* a montré que l'hirudine synthétisée par *E. coli* reste intracellulaire et doit donc être purifiée à partir d'un très grand nombre de polypeptides de *E. coli*. Il est donc particulièrement intéressant de pouvoir faire exprimer un gène de l'hirudine dans la levure de façon à obtenir une hirudine sécrétée sous forme mature, et sans que la levure ne produise de substances pyrogènes ou toxiques pour l'homme.

L'invention s'applique donc à toutes les molécules d'hirudines, c'est à dire, variants naturels de l'hirudine tel quels ou ayant subi une ou plusieurs mutations tout en conservant leur activité antithrombotique, ce dernier type de variant étant appelé analogue. Les exemples ci-après concerneront plus particulièrement l'analogue désigné par rHV2Lys47 (pour recombinant variant HV2 ayant subi une mutation de l'acide aminé Asp en position 47 en acide aminé Lys), décrit dans la publication de brevet EP-A-02738000 déjà mentionnée.

L'invention s'applique également à la production de défensines. Les défensines, aussi appelées phormicines, sont des peptides originellement extraits de l'hémolymphe des certains insectes, les Diptères, qui ont une activité bactéricide sur les germes Gram-positifs. Ces défensines sont plus amplement décrites dans la demande de brevet européenne EP-A-349 451. La défensine A est un peptide basique ayant pour séquence

Ala Thr Cys Asp Leu Leu Ser Gly Thr

Gly Ile Asn His Ser Ala Cys Ala Ala His Cys Leu Leu Arg Gly Asn Arg Gly Gly

Tyr Cys Asn Gly Lys Gly Val Cys Val Cys Arg Asn.

La défensine B ne diffère de la défensine A que par l'acide aminé en position 32 où une arginine remplace une glycine.

Les exemples ci-après permettront de mettre en évidence d'autres caractéristiques et avantages de la présente invention. Ces exemples seront illustrés par les figures suivantes :
- la figure 1 représente la structure schématique du plasmide pTG2958.
- la figure 2 représente la structure schématique des vecteurs M13TG3839 et M13TG3841. Pour M13TG3839 les cadres hachurés aux extrémités correspondent à M13TG103 et pour M13TG3841 à M13TG3149.
- la figure 3 représente la structure schématique du vecteur M13TG3845. Le cadre hachuré correspond à M13TG3149.
- la figure 4 représente la structure schématique du plasmide pTG3828.
- la figure 5 représente la structure schématique du plasmide pTG3864.

EXEMPLE 1 : Construction des vecteurs d'expression de l'hirudine : pTG3864, pTG3867, pTG3894 et pTG3884.

A. Construction du vecteur M13TG3845

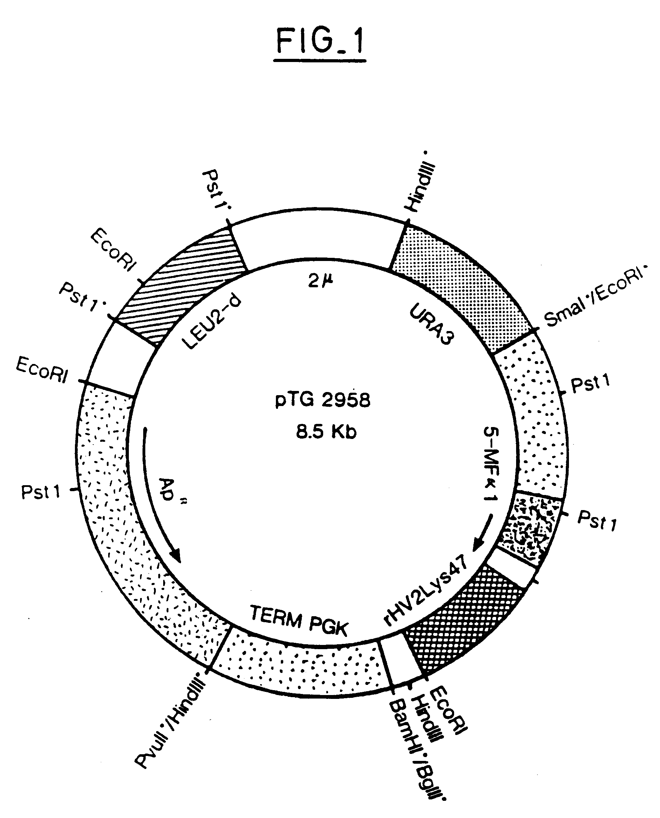

Le plasmide pTG2958 (figure 1) est peu différent du plasmide pTG1833 décrit dans la publication européenne de brevet EP-A-252854 porteur de la séquence codante pour rHV2Asp47. Le plasmide pTG2958 ne contient pas le site de restriction HindIII artificiellement introduit. Le plasmide pTG2958 contient :
- un fragment de 1217 paires de bases correspondant à la région 5' du gène MFα1 (contenant le promoteur, la séquence codant pour le peptide signal, la région "pro" et une séquence codant pour le

8

peptide Lys-Arg), et 4 paires de bases (site BglII°, traitement par Klenow),
- un fragment de 234 paires de bases contenant l'ADN complémentaire de rHV2Lys47,
- un fragment de 243 paires de bases comprenant le terminateur PGK de levure,
- le fragment PvuII-EcoRI de pBR322 comprenant entre autres l'origine de réplication de ce plasmide et le gène de résistance à l'ampicilline (2292 paires de bases),
- le fragment EcoRI-HindIII du plasmide 2μ de la levure (forme B), contenant le gène LEU2 de levure, sous forme délétée et inséré dans le site PstI,
- un fragment HindIII-SmaI du gène URA3 de levure.

Le fragment NcoI-NcoI du vecteur pTG2958 qui porte les séquences LEU2-d, 2μ et URA3 est remplacé par le fragment NcoI-NcoI de pTG2800 décrit dans la publication européenne de brevet EP-A-O268501 qui porte les séquences du plasmide 2μ et du gène URA3 délété de son promoteur (URA3-d) pour donner pTG2877.

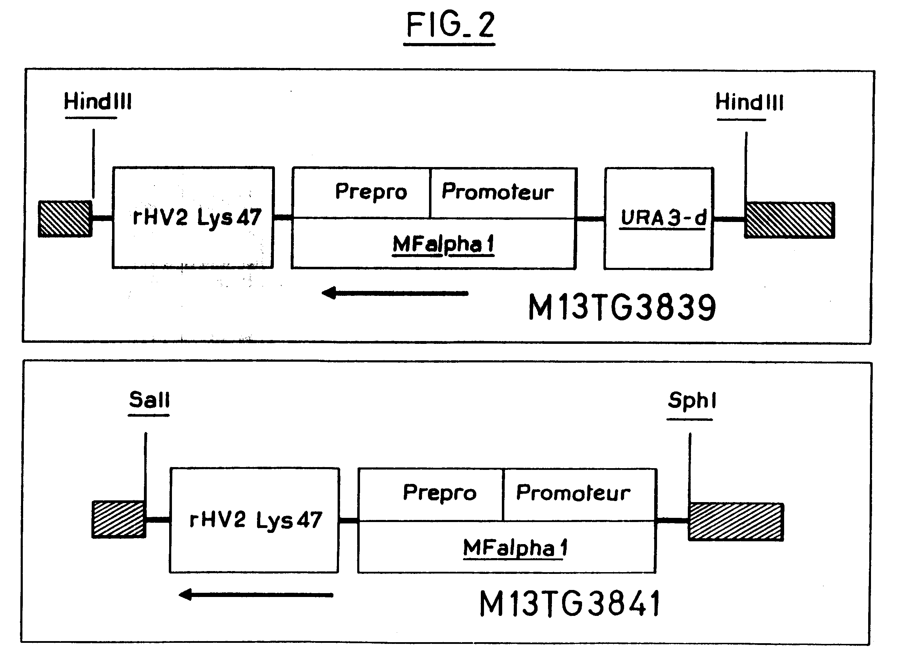

Le vecteur M13TG3839 (figure 2) dérive de M13TG103 [Kieny, M.P. *et al.* (1983) Gene 26, 91-99] dans lequel le fragment HindIII-HindIII de pTG2877 est introduit dans le même site. Un site de restriction SalI est introduit dans ce vecteur en aval du codon de terminaison de traduction de la région codant pour rHV2Lys47 par mutagénèse dirigée à l'aide de l'oligonucléotide suivant :
5' CAATGAAAAATGGTCGACTATCAATCATAG pour donner M13TG3839 SalI. Un site de restriction SphI est alors introduit en amont de la cassette d'expression en éliminant la séquence URA3-d par mutagénèse dirigée à l'aide de l'oligonucléotide suivant :
5' GACGGCCAGTGAATTGGCATGCTATTGATAAGATTTAAAG pour donner M13TG3840.

Le vecteur M13TG131 [Kieny M.P. *et al.* (1983) Gene 26, 91-99] est clivé par PstI, les extrémités rendues franche par traitement à l'aide du fragment Klenow de l'ADN polymérase I pour être ensuite religué sur lui même pour donner M13TG3160. Ce vecteur est ensuite clivé par SmaI et EcoRV puis religué pour donner M13TG3149.

Le fragment SphI-SalI de M13TG3840 (décrit ci-dessus) portant la cassette d'expression de rHV2Lys47 (sans séquence terminatrice de transcription) est introduit dans le site SphI-SalI de M13TG3149 pour donner M13TG3841 (figure 2).

La séquence codant pour les acides aminés en position 3 à 42 sur le fragment "pro" du précurseur de MFα1 est éliminée de M13TG3149 par mutagénèse dirigée et un site de restriction SmaI introduit en utilisant l'oligonucléotide suivant :
5' CTCCGCATTAGCTGCTCCCGGGGTTATTGTTTATAAAT, pour donner ce que l'on appelle par la suite une sequence "pro" délétée.

On obtient ainsi M13TG3842. Un site de restriction BamHI détruisant l'ATG du précurseur du facteur α est introduit dans ce vecteur par mutagénèse dirigée avec l'oligonucléotide suivant :
5' AATATAAACGATTAAAAGGATCCGATTTCCTTCAATTTTTA

On obtient alors M13TG3843. Après phosphorylation, les oligonucléotides suivants:

```
5' GATCCGTTTCTCTACTACAGTCGCTACTGCAGCTACTGCGCTATT
        GCAAAGTGATGATGTCAGCGATG 5'

et
                5' TTTCACAGCCTCCCAAGTTTCAGCTGCTCCC
    ACGTCGATGACGCGATAAAAAGTGTCGGAGGGTTCAAAGTCGACGAGGG 5'
```

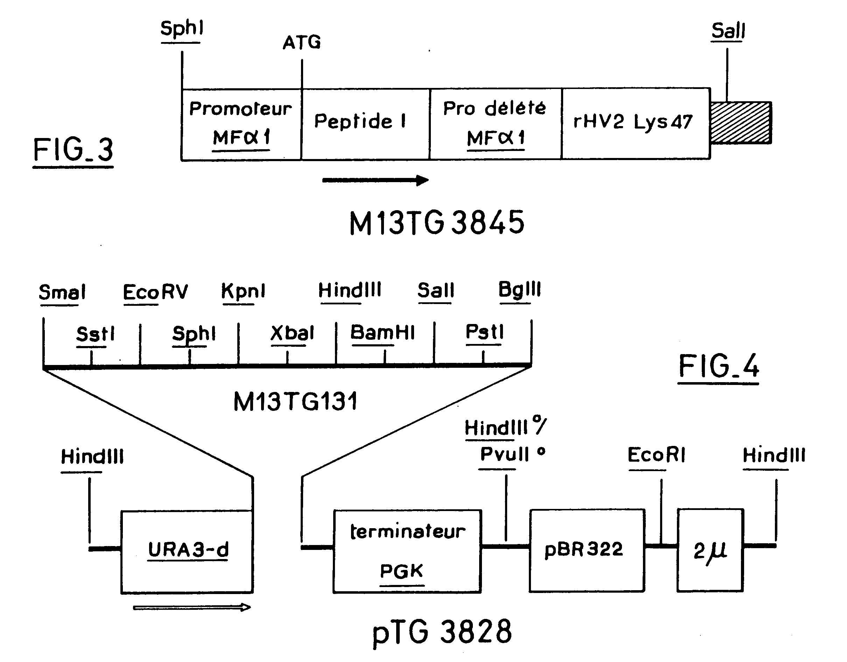

sont insérés dans le vecteur M13TG3843 coupé par BamHI et SmaI introduisant ainsi la séquence XI donc sans ATG. De manière à restaurer l'ATG, le site BamHI est éliminé par mutagénèse dirigée en utilisant l'oligonucléotide suivant :
5' AATATAAACGATTAAAAGAATGCGTTTCTCTACTACAGTC
pour donner le vecteur M13TG3845 (figure 3) qui comporte :
- le promoteur du gène MFα1, suivi d'un codon ATG, en tant que fragment a)
- la séquence XI en tant que fragment b),
- la séquence "pro" délétée du gène MFα1, suivi des codons codant pour Lys-Arg en tant que fragment b'),
- la séquence codant pour rHV2Lys47 en tant que fragment c),
- une partie du vecteur M13TG3149.

B. Construction du plasmide pTG3864

Le plasmide pTG848 décrit dans la publication européenne de brevet EP-A-0252854 est digéré par BglII puis religué pour donner pTG2886. Le grand fragment HindIII-EcoRI de pTG2886 est ligué en présence de ligase T4 au fragment HindIII-EcoRI de 2,1 kb du plasmide pFL1 [Parent, S.A. *et al.* (1985) Yeast 1, 83-138] qui porte la séquence du plasmide 2μ de *S. cerevisiae* pour donner le plasmide pTG2886 LEU2-d, URA3-d. Le fragment HindIII de 0.9 kb du plasmide pTG2800 décrit dans la publication européenne de brevet EP-A-0258501 portant le gène URA3-d est alors inséré dans le site HindIII de ce plasmide pour donner pTG2886 URA3-d, delta LEU2-d. Le fragment SmaI-BglII de M13TG131 [Kieny *et al.* (1983) Gene 26, 91-99] qui possède plusieurs sites de restriction est ensuite introduit dans ce plasmide pour donner pTG3828 (figure 4) qui comporte:

- la séquence du gène URA3 délétée de son promoteur (URA3-d),
- des sites de restriction venant de M13TG131 permettant l'insertion des éléments de l'expression d'un gène hétérologue, rHV2Lys47 dans le cas présent,
- le terminateur de transcription du gène PGK de levure
- un fragment de pBR322 qui permet la réplication et la sélection chez *E. coli*,
- un fragment du plasmide 2μ qui possède les éléments structuraux nécessaires à la réplication et à l'équipartition mitotique dans la levure.

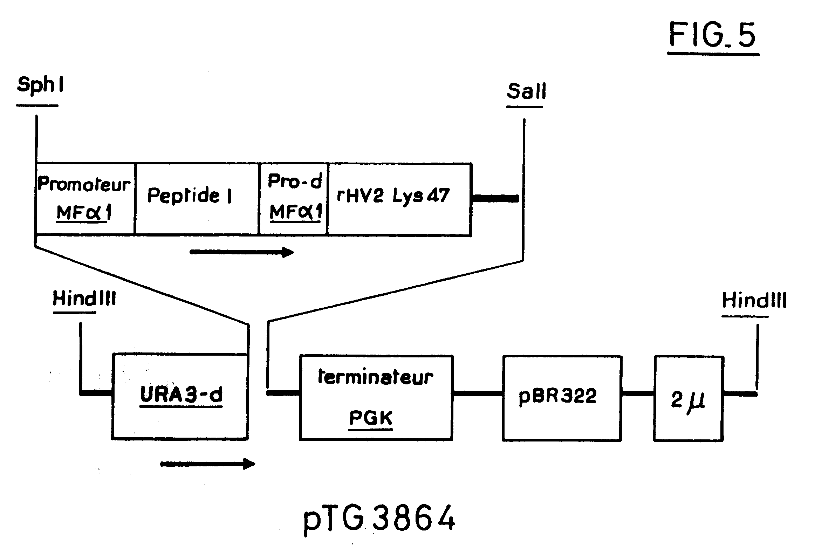

Le fragment SphI-SalI du vecteur M13TG3845 (figure 3) est introduit dans le plasmide pTG3828 digéré par SphI et SalI pour donner le vecteur d'expression pTG3864 (figure 5).

C. Construction du vecteur d'expression pTG3867

Pour supprimer complètement la séquence codant pour le précurseur du gène MFα1 on effectue une mutagénèse dirigée sur M13TG3845 (figure 3) à l'aide de l'oligonucléotide suivant :

5' GCCTCCCAAGTTTCAGCTATTACGTATACAGACTGC

pour obtenir le vecteur M13TG3846. Le fragment SphI-SalI de ce vecteur est introduit dans le plasmide pTG3828 (figure 4) digéré par SphI et SalI pour donner le vecteur d'expression pTG3867. Dans ce vecteur, la séquence XI et celle codant pour rHV2Lys47 sont adjacentes. Pour obtenir la structure schématique de ce plasmide, il suffit sur la figure 5 de retirer la séquence "pro" délétée de MFα1.

D. Construction du vecteur d'expression pTG3894

Un site SmaI est créé dans la séquence codant pour le fragment "pro" du précurseur du facteur α par mutagénèse dirigée sur M13TG3841 (figure 2) grâce à l'oligonucléotide suivant :

5' TCCGCATTAGCTGCTCCCGGGAACACTACAACAGAA

pour obtenir M13TG3869. Ce vecteur est ensuite digéré par SphI et SmaI, le petit fragment est isolé et il est ligué au grand fragment SphI et SmaI de M13TG3845 (figure 3) pour donner le vecteur M13TG3891. Le fragment SphI-SalI de ce vecteur est introduit dans le plasmide pTG3828 (figure 4) ouvert aux sites SphI et SalI pour donner le vecteur d'expression pTG3894 qui comporte donc la séquence "pro" du précurseur du facteur α mutée. Pour obtenir la structure schématique de ce plasmide, il suffit sur la figure 5 de remplacer la séquence "pro" délétée de MFα1 par la séquence "pro" mutée.

E. Construction du vecteur d'expression pTG3884

La séquence XI est modifiée par mutagénèse dirigée sur M13TG3845 en utilisant l'oligonucléotide suivant :

5' GTTTCTCTACTACACTCGCTACTGC

La modification d'une seule base donne la séquence XII et induit le remplacement d'une valine par une leucine en tant qu'acide aminé $R_4$ du peptide signal. On obtient ainsi le bactériophage M13TG3846. Le fragment SphI-SalI de M13TG3846 est introduit dans le plasmide pTG3828 (figure 4) ouvert aux sites SphI et SalI pour donner le vecteur d'expression pTG3884 qui comporte :

- la séquence du gène URA3 délétée de son promoteur (URA3-d),
- le promoteur du gène MFα1, suivi d'un codon ATG, en tant que fragment a),
- la séquence XII en tant que fragment b),
- la séquence "pro" délétée du gène MFα1, suivi des codons codant pout Lys-Arg, en tant que fragment b'),
- la séquence codant pour rHV2Lys47 en tant que fragment c),

- le terminateur du gène codant pour PGK de la levure.
- un fragment de pBR322,
- un fragment du plasmide 2μ.

EXEMPLE 2 : Production de rHV2Lys47 dans le surnageant de culture en fonction du plasmide utilisé.

Une souche de levure de l'espèce *Saccharomyces cerevisiae* de génotype MATα, ura3-251,-373,-328, leu2-3,-112, his3, pep4-3 est transformée par les plasmides pTG3864, pTG3867, pTG3894 et pTG3884 par le méthode de l'acétate de lithium [Ito, H. *et al.* J. Bactériol. (1983) 153: 163] et les prototrophes Ura + sont sélectionnés. Ils sont ensuite mis en culture en erlenmeyer à 30°C sur un milieu sélectif (0,7% de bases azotées pour levures (Yeast Nitrogen Base), 0,5% de casamino acides et 1% de glucose). Après 48 heures de culture, on sépare cellules et surnageant par centrifugation et l'activité inhibitrice de la thrombine est déterminée dans le surnageant en utilisant le test colorimétrique (activité protéolytique sur un substrat synthétique, le chromozyme TH - Boehringer Mannheim). Le tableau I présente les résultats des dosages; chaque valeur correspond à la moyenne de deux expériences indépendantes. L'activité de rHV2Lys47 est exprimée en ATU/ml de surnageant.

Tableau I

| Plasmide | ATU/ml |
|----------|--------|
| pTG3894  | 40     |
| pTG3864  | 50     |
| pTG3867  | 130    |
| pTG3884  | 125    |

Dans tous les cas on mesure une activité anti-thrombine. La protéine rHV2Lys47 produite par la levure est donc excrétée dans le surnageant. De plus elle est secrétée sous forme active. Les meilleurs résultats sont obtenus pour les souches transformées par pTG3884 et pTG3867.

Le contenu en protéine des surnageants est analysé par HPLC. Le pic majeur obtenu correspond bien à celui de rHV2Lys47 (sous sa forme à 65 acides aminés) et la détermination de la séquence N-terminale confirme l'obtention d'une molécule correctement synthétisée.

EXEMPLE 3 : Construction d'un vecteur d'expression de la défensine A d'insectes : pTG4826.

A. Synthèse d'une séquence d'ADN codant pour la défensine A d'insecte.

La synthèse se fait en deux blocs assemblés grâce à leurs extrémités cohésives KpnI. Le premier bloc comprend 3 oligonucléotides numérotés de 1 à 3 et le second bloc, 6 oligonucléotides numérotés de 4 à 9. Leur séquence et la position des oligonucléotides (dernière ligne du tableau; les ronds représentent la partie 5' de l'oligonucléotide) sont donnés dans le tableau II.

EP 0 423 302 B1

## TABLEAU II

| n° | Séquence |
|---|---|
| 1 | 5' AGCTTGGACAAGAGAGCTACCTGTGACTTGTTGTCCGGTAC |
| 2 | 5' GGTAGCTCTCTTGTCCA |
| 3 | 5' CGGACAACAAGTCACA |
| 4 | 5' CGGTATTAACCACTCCGCTTGTGCTGCTCACTGTTTGTTG |
| 5 | 5' AGCACAAGCGGAGTGGTTAATACCGGTAC |
| 6 | 5' AGAGGTAACAGAGGTGGCTACTGTAACGGTAAGGGTGT |
| 7 | 5' AGTAGCCACCTCTGTTACCTCTCAACAAACAGTGAGC |
| 8 | 5' TTGTGTTTGTAGAAACTAAGGATCCG |
| 9 | 5'AATTCGGATCCTTAGTTTCTACAAACACAAACACCCTTACCGT TAC |

```
      1            4            6            8
   o────────    o────────   o────────    o────────
     ────o  ────o   ────o    ────o   ────o   ────o
      2     3       5        7        9
```

La séquence obtenue est la suivante :

```
HindIII                    +1                              KpnI
10
AGC TTG GAC AAG AGA GCT ACC TGT GAC TTG TTG TCC GGT ACC
                    Ala Thr Cys Asp Leu Leu Ser Gly Thr

                                            20
GGT ATT AAC CAC TCC GCT TGT GCT GCT CAC TGT TTG TTG AGA
Gly Ile Asn His Ser Ala Cys Ala Ala His Cys Leu Leu Arg
```

```
                              30
GGT AAC AGA GGT GGC TAC TGT AAC GGT AAG GGT GTT TGT GTT
Gly Asn Arg Gly Gly Tyr Cys Asn Gly Lys Gly Val Cys Val

         40              BamHI-EcoRI
TGT AGA AAC TAA GGATCCG
Cys Arg Asn
```

La synthèse du premier bloc utilise les oligonucléotides 4, 5, 6, 7, 8 et 9 et s'effectue de la façon suivante :

- les oligonucléotides 5, 6, 7 et 8 sont tout d'abord phosphorylés à leur extrémités 5' pour éviter la formation de polymères au cours de l'assemblage. Pour chacun de ces oligonucléotides, 100 picomoles sont traitées à la polynucléotide kinase, 2 unités dans un volume final de 20 $\mu$l de Tris HCl 60 $\mu$M à pH 7,5; 10 $\mu$M de $MgCl_2$; 8 $\mu$M de dithiothréitol (tampon de kination) contenant 3,3 picomoles d'ATP$\gamma$ marqué avec $^{32}$P (5000 Ci/mmole). Après 15 minutes d'incubation à 37°C, 5 $\mu$moles d'ATP non marqué sont ajoutées.
- après incubation à 37°C pendant 30 min. 75 picomoles des oligonucléotides 5, 6, 7 et 8 sont mélangés, chauffés à 95°C pendant 3 min., puis les oligonucléotides 4 et 9 sont ajoutés dans un volume final de 90 $\mu$moles de tampon de kination décrit ci-dessus. L'ensemble est chauffé à 95°C pendant 3 min. puis refroidi lentement en 2 heures à 37°C.
- 25 picomoles de ces oligonucléotides hybridés sont soumis au traitement par la ligase T4 pendant une heure à 15°C. Ce mélange réactionnel (1 picomole) est ensuite ajouté à 50 ng du du bactériophage M13TG131 [Kieny M.P. *et al*. (1983) Gene 26, 91-99] traité par EcoRI et KpnI (1 heure à 15°C). Le mélange de ligation est utilisé pour transformer les cellules compétentes de la souche *E. coli* JM103 [Messing J. *et al*. (1981), Nucleic Acid Res. 9, 309]. Un clone présentant la séquence recherchée est isolé, il est appelé M13TG3821.

La synthèse du second bloc utilise les oligonucléotides 1, 2 et 3 et s'effectue selon la même procédure que celle décrite pour la synthèse du premier bloc. Dans ce cas, seul l'oligonucléotide 2 est phosphorylé à son extrémité 5'.

Ce second bloc est cloné entre les sites HindIII et KpnI du bactériophage M13TG3821 et un clone portant la séquence d'ADN codant pour la défensine A (figure 5) est isolé, il est appelé M13TG3849.

B. Construction du plasmide d'expression de la défensine A: pTG4839.

Le fragment SphI - SmaI de 1045 paires de bases du bactériophage M13TG3846 décrit précédemment (Exemple 1, E.) est transféré dans le vecteur M13TG3869 décrit précédemment (Exemple 1, D.) préalablement digéré par SphI et SmaI. On obtient ainsi le vecteur M13TG4803 qui porte :
- le promoteur du gène MF$\alpha$1, suivi d'un codon ATG,
- la séquence XII,
- la séquence "pro" mutée du gène MF$\alpha$1 suivi des codons codant pour Lys-Arg,
- la séquence codant pour rHV2Lys47.

Afin de remplacer la séquence codant pour rHV2Lys47 par celle codant pour la défensine A d'insecte on introduit un site HindIII dans la séquence codant pour "pro" mutée de MF$\alpha$1 à l'aide de l'oligonucléotide de séquence :
5' GAAGGGGGTAAGCTTGGATAAA
Puis on introduit le fragment HindIII - BamHI de M13TG3849 décrit précédemment (Exemple 3, A.) qui porte la séquence synthétique codant pour la défensine A dans ce vecteur préalablement traité par HindIII et BamHI pour éliminer la séquence codant pour rHV2Lys47.

Le fragment SphI-SalI de M13TG4813 est introduit dans le plasmide pTG3828 (figure 4) ouvert aux sites SphI et SalI pour donner le vecteur d'expression pTG4839 qui comporte :
- la séquence du gène URA3 délétée de son promoteur (URA3-d),
- le promoteur du gène MF$\alpha$1, suivi d'un ATG,
- la séquence XII,
- la séquence "pro" mutée du gène MF$\alpha$1 suivi des codons codant pour Lys-Arg,
- la séquence synthétique codant pour la défensine A d'insectes,
- le terminateur du gène codant pour PGK de la levure.
- un fragment de pBR322,

- un fragment du plasmide 2μ.

EXEMPLE 4 : Production de défensine A dans le surnageant de culture.

Une souche de levure de l'espèce *Saccharomyces cerevisiae* de génotype MATα, ura3-251,-373,-328, leu2-3,-112, his3, pep4-3 est transformée par le plasmide pTG4839 par la méthode de l'acétate de lithium [Ito, H. *et al*. J. Bactériol. (1983) 153: 163] et les prototrophes Ura + sont sélectionnés. Ils sont ensuite mis en culture en erlenmeyer à 30°C sur un milieu sélectif (0,7% de bases azotées pour levures (Yeast Nitrogen Base), 0,5% de casamino acides et 1% de glucose). Après 48 heures de culture, on sépare cellules et surnageant par centrifugation et le surnageant est filtré sur un filtre de 22 μ puis passé sur cartouche Sep-Pak C18. Le matériel fixé est élué avec 60% d'acétonitrile, 0,1% d'acide trifluoro acétique dans de l'eau et séché sous vide. L'activité antibactérienne de la défensine A est ensuite mise en évidence par un test d'étalement sur agar ou sur gélose ensemencé de germes bactériens (*Micrococcus luteus*) conformément à la procédure décrite par Lambert *et al*. (1989) PNAS 86: 262-266.

Dans le surnageant des levures transformées par le plasmide pTG4839 on détecte effectivement une activité antibactérienne. La protéine défensine A produite par la levure est donc excrétée dans le surnageant. De plus elle est secrétée sous forme active.

Le contenu en protéine des surnageants est analysé par HPLC. Le pic majeur obtenu correspond bien à celui de la défensine A d'insecte et la détermination de la séquence de la protéine confirme l'obtention d'une molécule correctement synthétisée.

**Revendications**
**Revendications pour les Etats contractants suivants : AT, BE, CH, DE, DK, FR, GB, IT, LI, LU, NL, SE**

1. Un fragment d'ADN isolé qui code pour un peptide signal utile pour la sécrétion d'une protéine hétérologue par une cellule dans laquelle la protéine hétérologue est synthétisée, dont la séquence d'acides aminés présente un degré d'homologie d'au moins 60% avec la séquence d'acides aminés de formule (I) ou (II)

    (I)     Arg-Phe-Ser-Thr-Thr-Leu-Ala-Thr-Ala-Ala-Thr-Ala-Leu-Phe-Phe-Thr-Ala-Ser-Gln et

    (II)   Arg-Phe-Ser-Thr-Thr-Leu-Ala-Thr-Ala-Ala-Thr-Ala-Leu-Phe-Phe-Thr-Ala-Ser-Gln-Val-Ser-Ala.

2. Un fragment d'ADN selon la revendication 1 qui code pour un peptide dont la séquence d'acides aminés présente un degré d'homologie d'au moins 80% avec la séquence d'acides aminés (I) ou (II) de formule

    (I)     Arg-Phe-Ser-Thr-Thr-Leu-Ala-Thr-Ala-Ala-Thr-Ala-Leu-Phe-Phe-Thr-Ala-Ser-Gln et

    (II)   Arg-Phe-Ser-Thr-Thr-Leu-Ala-Thr-Ala-Ala-Thr-Ala-Leu-Phe-Phe-Thr-Ala-Ser-Gln-Val-Ser-Ala.

3. Un fragment d'ADN selon la revendication 1 ou 2 qui code pour un peptide comprenant la séquence d'acides aminés (III)

(III) $R_1$-$R_2$-$R_3$-Thr-Thr-$R_4$-Ala-Thr-Ala-Ala-Thr-Ala-Leu-Phe-

Phe-Thr-Ala-$R_5$-$R_6$

dans laquelle :

$R_1$ est un acide aminé sélectionné parmi Arg et Lys,

$R_2$ et $R_6$ sont chacun un acide aminé sélectionné de manière indépendante parmi Ala, Asn, Cys, Gln, Gly, His, Ile, Leu, Met, Phe, Pro, Ser, Thr, Trp, Tyr et Val $R_3$ et $R_5$ sont chacun un acide aminé sélectionné de manière indépendante parmi Asp, Gly, Asn, Pro et Ser, et

$R_4$ est un acide aminé sélectionné parmi Val, Leu, Ala, Cys. Phe, Ile et Met.

4. Un fragment d'ADN selon la revendication 1 ou 2 qui code pour un peptide comprenant la séquence d'acides aminés (IV)

(IV)    $R_1$-$R_2$-$R_3$-Thr-Thr-$R_4$-Ala-Thr-Ala-Ala-Thr-Ala-Leu-Phe-Phe-Thr-

Ala-$R_5$-$R_6$-$R_7$

dans laquelle:

$R_1$ est un acide aminé sélectionné parmi Arg et Lys,

$R_2$ et $R_6$ sont chacun un acide aminé sélectionné de manière indépendante parmi Ala, Asn, Cys, Gln, Gly, His, Ile, Leu, Met, Phe, Pro, Ser, Thr, Trp, Tyr et Val

$R_3$ et $R_5$ sont chacun un acide aminé sélectionné de manière indépendante parmi Asp, Gly, Asn, Pro et Ser,

$R_4$ est un acide aminé sélectionné parmi Val, Leu, Ala, Cys, Phe, Ile et Met, et $R_7$ est une séquence de protéolyse.

5. Un fragment d'ADN selon la revendication 4 dans lequel $R_7$ est une séquence de protéolyse $R_8$-$R_9$-$R_{10}$ dans laquelle:

$R_8$ est un acide aminé sélectionné parmi Ala, Val, Ser, Cys, Gly, Ile, Leu, Thr,

$R_9$ est un acide aminé sélectionné parmi Ala, Arg, Cys, Gln, Gly, His, Ile, Leu, Met, Phe, Pro, Ser, Thr, Trp, Tyr et Val et

$R_{10}$ est un acide aminé sélectionné parmi Ala, Cys, Gly, Leu, Pro, Gln, Ser et Thr.

6. Un fragment d'ADN selon la revendication 1 ou 2 qui code pour un peptide comprenant une séquence d'acides aminés sélectionnée parmi les séquences d'acides amines (V) et (VI)

(V)    Arg-Phe-Ser-Thr-Thr-Leu-Ala-Thr-Ala-Ala-Thr-Ala-Leu-Phe-Phe-Thr-

Ala-Ser-Gln;

(VI)    Arg-Phe-Ser-Thr-Thr-Val-Ala-Thr-Ala-Ala-Thr-Ala-Leu-Phe-Phe-Thr-

Ala-Ser-Gln.

7. Un fragment d'ADN selon l'une des revendications 1, 2, 4 et 5 qui code pour un peptide comprenant une séquence d'acides aminés sélectionnée parmi les séquences d'acides aminés de formule (VII) et (VIII)

15

(VII) Arg-Phe-Ser-Thr-Thr-Leu-Ala-Thr-Ala-Ala-Thr-Ala-Leu-Phe-Phe-Thr-Ala-Ser-Gln-$R_7$

dans laquelle $R_7$ est une séquence de protéolyse;

(VIII) Arg-Phe-Ser-Thr-Thr-Val-Ala-Thr-Ala-Ala-Thr-Ala-Leu-Phe-Phe-Thr-Ala-Ser-Gln-$R_7$

dans laquelle $R_7$ est une séquence de protéolyse.

8. Un fragment d'ADN selon la revendication 7 qui code pour un peptide comprenant une séquence d'acides aminés sélectionnée parmi les séquences d'acides aminés de formule (VII) et (VIII) dans lesquelles $R_7$ est Val-Ser-Ala.

9. Application d'un fragment d'ADN selon l'une des revendications 1 à 8, caractérisé en ce que le fragment d'ADN code pour un peptide signal utile pour la sécrétion d'une protéine hétérologue par une cellule de levure dans laquelle la protéine hétérologue est synthétisée.

10. Une cassette d'expression d'une protéine hétérologue comprenant au moins :
     a) un fragment d'ADN comportant des signaux d'initiation de transcription et de traduction,
     b) un fragment d'ADN selon l'une des revendications 1 et 8 et,
     c) un fragment d'ADN codant pour la protéine hétérologue mature.

11. Une cassette selon la revendication 10 comprenant en outre un fragment d'ADN b') codant pour un fragment peptidique "pro".

12. Une cassette selon la revendication 11 comprenant un fragment d'ADN b') codant pour un fragment peptidique "pro" ayant pour séquence

Ala Pro Gly Leu Leu Phe Ile Asn Thr Thr Ile Ala Ser Ile Ala Ala Lys Glu Glu Gly Val Ser Leu Asp Lys Arg.

13. Une cassette selon l'une des revendications 10 à 12, caractérisée en ce que le fragment a) comporte un promoteur fonctionnel dans une cellule de levure et un codon d'initiation de traduction ATG.

14. Une cassette d'expression selon l'une des revendications 10 à 13, caractérisée en ce que le fragment d'ADN c) code pour une hirudine.

15. Une cassette d'expression selon la revendication 14, caractérisée en ce que le fragment d'ADN c) code pour le variant hirudine rHV2Lys47.

16. Une cassette d'expression selon l'une des revendications 10 à 13, caractérisée en ce que le fragment d'ADN c) code pour une défensine d'insectes.

17. Une cassette d'expression selon la revendication 16, caractérisée en ce que le fragment d'ADN c) code pour la défensine A.

18. Un vecteur plasmidique comprenant une cassette d'expression selon l'une des revendications 10 à 17.

19. Un vecteur plasmidique selon la revendication 18, caractérisé en ce qu'il comporte un fragment du plasmide $2\mu$ de levure.

**20.** Un vecteur plasmidique selon la revendication 18 ou 19, caractérisé en ce qu'il comporte, en tant que gène de sélection, le gène URA3 délété de son promoteur.

**21.** Une cellule transformée par un vecteur plasmidique selon l'une des revendications 18 à 20 ou ayant intégré dans son génome une cassette d'expression selon l'une des revendications 10 à 17.

**22.** Une cellule de levure selon la revendication 21.

**23.** Un procédé de préparation d'une protéine hétérologue, caractérisé en ce que l'on cultive une cellule selon la revendication 21 ou 22, et en ce que l'on récupère ladite protéine dans le milieu de culture.

**24.** Un procédé selon la revendication 23, caractérisé en ce que la cellule est une cellule de levure.

**25.** Un procédé selon la revendication 23 ou 24, caractérisé en ce que ladite protéine est une hirudine.

**26.** Un procédé selon la revendication 23 ou 24, caractérisé en ce que ladite protéine est une défensine d'insectes.

**Revendications pour l'Etat contractant suivant : ES**

**1.** Procédé de préparation d'un fragment d'ADN isolé qui code pour un peptide signal utile pour la sécrétion d'une protéine hétérologue par une cellule dans laquelle la protéine hétérologue est synthétisée, caractérisé en ce qu'on prépare un fragment d'ADN qui code pour un peptide dont la séquence d'acides aminés présente un degré d'homologie d'au moins 60% avec la séquence d'acides aminés de formule (I) ou (II)

(I)   Arg-Phe-Ser-Thr-Thr-Leu-Ala-Thr-Ala-Ala-Thr-Ala-Leu-Phe-Phe-Thr-Ala-Ser-Gln et

(II)   Arg-Phe-Ser-Thr-Thr-Leu-Ala-Thr-Ala-Ala-Thr-Ala-Leu-Phe-Phe-Thr-Ala-Ser-Gln-Val-Ser-Ala.

**2.** Procédé selon la revendication 1, caractérisé en ce que le fragment d'ADN code pour un peptide dont la séquence d'acides aminés présente un degré d'homologie d'au moins 80% avec la séquence d'acides aminés (I) ou (II) de formule

(I)   Arg-Phe-Ser-Thr-Thr-Leu-Ala-Thr-Ala-Ala-Thr-Ala-Leu-Phe-Phe-Thr-Ala-Ser-Gln et

(II)   Arg-Phe-Ser-Thr-Thr-Leu-Ala-Thr-Ala-Ala-Thr-Ala-Leu-Phe-Phe-Thr-Ala-Ser-Gln-Val-Ser-Ala.

**3.** Procédé selon l'une des revendications 1 ou 2, caractérisé en ce que le fragment d'ADN code pour un peptide comprenant la séquence d'acides aminés (III)

(III) $R_1$-$R_2$-$R_3$-Thr-Thr-$R_4$-Ala-Thr-Ala-Ala-Thr-Ala-Leu-Phe-
         1                5                                    10

Phe-Thr-Ala-$R_5$-$R_6$
       15                19

dans laquelle :

$R_1$ est un acide aminé sélectionné parmi Arg et Lys,

$R_2$ et $R_6$ sont chacun un acide aminé sélectionné de manière indépendante parmi Ala, Asn, Cys, Gln, Gly, His, Ile, Leu, Met, Phe, Pro, Ser, Thr, Trp, Tyr et Val,

$R_3$ et $R_5$ sont chacun un acide aminé sélectionné de manière indépendante parmi Asp, Gly, Asn, Pro et Ser, et

$R_4$ est un acide aminé sélectionné parmi Val, Leu, Ala, Cys, Phe, Ile et Met.

4. Procédé selon l'une des revendications 1 ou 2, caractérisé en ce que le fragment d'ADN code pour un peptide comprenant la séquence d'acides aminés (IV)

$$(IV) \quad R_1\text{-}R_2\text{-}R_3\text{-}Thr\text{-}Thr\text{-}R_4\text{-}Ala\text{-}Thr\text{-}Ala\text{-}Ala\text{-}Thr\text{-}Ala\text{-}Leu\text{-}Phe\text{-}Phe\text{-}Thr\text{-}Ala\text{-}R_5\text{-}R_6\text{-}R_7$$

dans laquelle :

$R_1$ est un acide aminé sélectionné parmi Arg et Lys,

$R_2$ et $R_6$ sont chacun un acide aminé sélectionné de manière indépendante parmi Ala, Asn, Cys, Gln, Gly, His, Ile, Leu, Met, Phe, Pro, Ser, Thr, Trp, Tyr et Val,

$R_3$ et $R_5$ sont chacun un acide aminé sélectionné de manière indépendante parmi Asp, Gly, Asn, Pro et Ser,

$R_4$ est un acide aminé sélectionné parmi Val, Leu, Ala, Cys, Phe, Ile et Met, et

$R_7$ est une séquence de protéolyse.

5. Procédé selon la revendication 4 dans lequel $R_7$ est une séquence de protéolyse $R_8\text{-}R_9\text{-}R_{10}$ dans laquelle :

$R_8$ est un acide aminé sélectionné parmi Ala, Val, Ser, Cys, Gly, Ile, Leu, Thr,

$R_9$ est un acide aminé sélectionné parmi Ala, Arg, Cys, Gln, Gly, His, Ile, Leu, Met, Phe, Pro, Ser, Thr, Trp, Tyr et Val et

$R_{10}$ est un acide aminé sélectionné parmi Ala, Cys, Gly, Leu, Pro, Gln, Ser et Thr.

6. Procédé selon l'une des revendications 1 ou 2, caractérisé en ce que le fragment d'ADN code pour un peptide comprenant une séquence d'acides aminés sélectionnée parmi les séquences d'acides aminés (V) et (VI)

$$(V) \quad Arg\text{-}Phe\text{-}Ser\text{-}Thr\text{-}Thr\text{-}Leu\text{-}Ala\text{-}Thr\text{-}Ala\text{-}Ala\text{-}Thr\text{-}Ala\text{-}Leu\text{-}Phe\text{-}Phe\text{-}Thr\text{-}Ala\text{-}Ser\text{-}Gln;$$

$$(VI) \quad Arg\text{-}Phe\text{-}Ser\text{-}Thr\text{-}Thr\text{-}Val\text{-}Ala\text{-}Thr\text{-}Ala\text{-}Ala\text{-}Thr\text{-}Ala\text{-}Leu\text{-}Phe\text{-}Phe\text{-}Thr\text{-}Ala\text{-}Ser\text{-}Gln.$$

7. Procédé selon l'une des revendications 1, 2, 4, ou 5, caractérisé en ce que le fragment d'ADN code pour un peptide comprenant une séquence d'acides aminés sélectionnée parmi les séquences d'acides aminés de formule (VII) et (VIII)

$$(VII) \quad Arg\text{-}Phe\text{-}Ser\text{-}Thr\text{-}Thr\text{-}Leu\text{-}Ala\text{-}Thr\text{-}Ala\text{-}Ala\text{-}Thr\text{-}Ala\text{-}Leu\text{-}Phe\text{-}Phe\text{-}Thr\text{-}Ala\text{-}Ser\text{-}Gln\text{-}R_7$$

dans laquelle $R_7$ est une séquence de protéolyse;

(VIII) Arg-Phe-Ser-Thr-Thr-Val-Ala-Thr-Ala-Ala-Thr-Ala-Leu-Phe-Phe-Thr-Ala-Ser-Gln-R,

dans laquelle R$_7$ est une séquence de protéolyse.

8.  Procédé selon la revendication 7, caractérisé en ce que le fragment d'ADN code pour un peptide comprenant une séquence d'acides aminés sélectionnée parmi les séquences d'acides aminés de formule (VII) et (VIII) dans lesquelles R$_7$ est Val-Ser-Ala.

9.  Application d'un fragment d'ADN préparé selon l'une des revendications 1 à 8, caractérisé en ce que le fragment d'ADN code pour un peptide signal utile pour la sécrétion d'une protéine hétérologue par une cellule de levure dans laquelle la protéine hétérologue est synthétisée.

10. Procédé de préparation d'une cassette d'expression d'une protéine hétérologue, caractérisé en ce qu'on prépare une cassette comprenant au moins :
    a) un fragment d'ADN comportant des signaux d'initiation de transcription et de traduction,
    b) un fragment d'ADN selon l'une des revendications 1 et 8 et,
    c) un fragment d'ADN codant pour la protéine hétérologue mature.

11. Procédé de préparation d'une cassette selon la revendication 10, ladite cassette comprenant en outre un fragment d'ADN b') codant pour un fragment peptidique "pro".

12. Procédé de préparation d'une cassette selon la revendication 11, ladite cassette comprenant un fragment d'ADN b') codant pour un fragment peptidique "pro" ayant pour séquence

Ala Pro Gly Leu Leu Phe
Ile Asn Thr Thr Ile Ala Ser Ile Ala Ala Lys Glu Glu Gly Val Ser Leu Asp Lys
Arg.

13. Procédé de préparation d'une cassette selon l'une des revendications 10 à 12, caractérisée en ce que le fragment a) comporte un promoteur fonctionnel dans une cellule de levure et un codon d'initiation de traduction ATG.

14. Procédé de préparation d'une cassette d'expression selon l'une des revendications 10 à 13, caractérisée en ce que le fragment d'ADN c) code pour une hirudine.

15. Procédé de préparation d'une cassette d'expression selon la revendication 14, caractérisée en ce que le fragment d'ADN c) code pour le variant hirudine rHV2Lys47.

16. Procédé de préparation d'une cassette d'expression selon l'une des revendications 10 à 13, caractérisée en ce que le fragment d'ADN c) code pour une défensine d'insectes.

17. Procédé de préparation d'une cassette d'expression selon la revendication 16, caractérisée en ce que le fragment d'ADN c) code pour la défensine A.

18. Procédé de préparation d'un vecteur plasmidique, caractérisé en ce qu'on introduit une cassette d'expression selon l'une des revendications 10 à 17.

19. Procédé de préparation d'un vecteur plasmidique selon la revendication 18, caractérisé en ce que ledit plasmide comporte un fragment du plasmide 2$\mu$ de levure.

20. Procédé de préparation d'un vecteur plasmidique selon la revendication 18 ou 19, caractérisé en ce que le plasmide comporte, en tant que gène de sélection, le gène URA3 délété de son promoteur.

19

**21.** Une cellule transformée par un vecteur plasmidique selon l'une des revendications 18 à 20 ou ayant intégré dans son génome une cassette d'expression selon l'une des revendications 10 à 17.

**22.** Une cellule de levure selon la revendication 21.

**23.** Un procédé de préparation d'une protéine hétérologue, caractérisé en ce que l'on cultive une cellule selon la revendication 21 ou 22, et en ce que l'on récupère ladite protéine dans le milieu de culture.

**24.** Un procédé selon la revendication 23, caractérisé en ce que la cellule est une cellule de levure.

**25.** Un procédé selon la revendication 23 ou 24, caractérisé en ce que ladite protéine est une hirudine.

**26.** Un procédé selon la revendication 23 ou 24, caractérisé en ce que ladite protéine est une défensine d'insectes.

**Claims**
**Claims for the following Contracting States : AT, BE, CH, DE, DK, FR, GB, IT, LI, LU, NL, SE**

**1.** An isolated DNA fragment which codes for a signal peptide useful for the secretion of a heterologous protein by a cell in which the heterologous protein is synthesized, whose amino acid sequence exhibits an at least 60% degree of homology with the amino acid sequence of formula (I) or (II)

```
(I)     Arg-Phe-Ser-Thr-Thr-Leu-Ala-Thr-Ala-Ala-Thr-Ala-
Leu-Phe-Phe-Thr-Ala-Ser-Gln and
(II)    Arg-Phe-Ser-Thr-Thr-Leu-Ala-Thr-Ala-Ala-Thr-Ala-
Leu-Phe-Phe-Thr-Ala-Ser-Gln-Val-Ser-Ala.
```

**2.** A DNA fragment according to Claim 1, which codes for a peptide whose amino acid sequence exhibits an at least 80% degree of homology with the amino acid sequence (I) or (II) of formula

```
(I)     Arg-Phe-Ser-Thr-Thr-Leu-Ala-Thr-Ala-Ala-Thr-Ala-
Leu-Phe-Phe-Thr-Ala-Ser-Gln and
(II)    Arg-Phe-Ser-Thr-Thr-Leu-Ala-Thr-Ala-Ala-Thr-Ala-
Leu-Phe-Phe-Thr-Ala-Ser-Gln-Val-Ser-Ala.
```

**3.** A DNA fragment according to Claim 1 or 2, which codes for a peptide comprising the amino acid sequence (III)

$$(III) \quad R_1\text{-}R_2\text{-}R_3\text{-}Thr\text{-}Thr\text{-}R_4\text{-}Ala\text{-}Thr\text{-}Ala\text{-}Ala\text{-}Thr\text{-}Ala\text{-}Leu\text{-}$$
$$\phantom{(III) \quad} 1 \phantom{xxxxxxxxxx} 5 \phantom{xxxxxxxxxxxxxxxxx} 10$$
$$\text{-}Phe\text{-}Phe\text{-}Thr\text{-}Ala\text{-}R_5\text{-}R_6$$
$$\phantom{xxxx} 15 \phantom{xxxxxxxxx} 19$$

in which:
$R_1$ is an amino acid selected from Arg and Lys,
$R_2$ and $R_6$ are each an amino acid selected independently from Ala, Asn, Cys, Gln, Gly, His, Ile, Leu, Met, Phe, Pro, Ser, Thr, Trp, Tyr and Val,
$R_3$ and $R_5$ are each an amino acid selected independently from Asp, Gly, Asn, Pro and Ser, and
$R_4$ is an amino acid selected from Val, Leu, Ala, Cys, Phe, Ile and Met.

4. A DNA fragment according to Claim 1 or 2, which codes for a peptide comprising the amino acid sequence

(IV)  $R_1$-$R_2$-$R_3$-Thr-Thr-$R_4$-Ala-Thr-Ala-Ala-Thr-Ala-Leu-Phe-Phe-Thr-Ala-$R_5$-$R_6$-$R_7$

in which:
$R_1$ is an amino acid selected from Arg and Lys,
$R_2$ and $R_6$ are each an amino acid selected independently from Ala, Asn, Cys, Gln, Gly, His, Ile, Leu, Met, Phe, Pro, Ser, Thr, Trp, Tyr and Val,
$R_3$ and $R_5$ are each an amino acid selected independently from Asp, Gly, Asn, Pro and Ser,
$R_4$ is an amino acid selected from Val, Leu, Ala, Cys, Phe, Ile and Met, and
$R_7$ is a proteolysis sequence.

5. A DNA fragment according to Claim 4, in which $R_7$ is a proteolysis sequence $R_8$-$R_9$-$R_{10}$, in which:
$R_8$ is an amino acid selected from Ala, Val, Ser, Cys, Gly, Ile, Leu and Thr,
$R_9$ is an amino acid selected from Ala, Arg, Cys, Gln, Gly, His, Ile, Leu, Met, Phe, Pro, Ser, Thr, Trp, Tyr and Val and
$R_{10}$ is an amino acid selected from Ala, Cys, Gly, Leu, Pro, Gln, Ser and Thr.

6. A DNA fragment according to Claim 1 or 2, which codes for a peptide comprising an amino acid sequence selected from the amino acid sequences (V) and (VI)

(V)  Arg-Phe-Ser-Thr-Thr-Leu-Ala-Thr-Ala-Ala-Thr-Ala-Leu-Phe-Phe-Thr-Ala-Ser-Gln;

(VI)  Arg-Phe-Ser-Thr-Thr-Val-Ala-Thr-Ala-Ala-Thr-Ala-Leu-Phe-Phe-Thr-Ala-Ser-Gln.

7. A DNA fragment according to one of Claims 1, 2, 4 and 5, which codes for a peptide comprising an amino acid sequence selected from the amino acid sequences of formula (VII) and (VIII)

(VII)  Arg-Phe-Ser-Thr-Thr-Leu-Ala-Thr-Ala-Ala-Thr-Ala-Leu-Phe-Phe-Thr-Ala-Ser-Gln-$R_7$,

in which $R_7$ is a proteolysis sequence:

(VIII)  Arg-Phe-Ser-Thr-Thr-Val-Ala-Thr-Ala-Ala-Thr-Ala-Leu-Phe-Phe-Thr-Ala-Ser-Gln-$R_7$

in which $R_7$ is a proteolysis sequence.

8. A DNA fragment according to Claim 7, which codes for a peptide comprising an amino acid sequence selected from the amino acid sequences of formula (VII) and (VIII) in which $R_7$ is Val-Ser-Ala.

9. Application of a DNA fragment according to one of claims 1 to 8, characterized in that the DNA fragment codes for a signal peptide useful for the secretion of a heterologous protein by a yeast cell in which the heterologous protein is synthesized.

10. A cassette for the expression of a heterologous protein, comprising at least:

21

a) a DNA fragment containing transcription and translation initiation signals,
b) a DNA fragment according to one of claims 1 to 8, and
c) a DNA fragment coding for the mature heterologous protein.

**11.** A cassette according to Claim 10, comprising, in addition, a DNA fragment b') coding for a "pro" peptide fragment.

**12.** A cassette according to Claim 11, comprising a DNA fragment b') coding for a "pro" peptide fragment having as its sequence

```
        Ala Pro Gly Leu Leu Phe Ile Asn
   Thr Thr Ile Ala Ser Ile Ala Ala Lys Glu Glu Gly Val Ser
   Leu Asp Lys Arg.
```

**13.** A cassette according to one of Claims 10 to 12, characterized in that the fragment a) contains a promoter which is functional in a yeast cell and an ATG translation initiation codon.

**14.** An expression cassette according to one of Claims 10 to 13, characterized in that the DNA fragment c) codes for a hirudin.

**15.** An expression cassette according to Claim 14, characterized in that the DNA fragment c) codes for the hirudin variant rHV2Lys47.

**16.** An expression cassette according to one of Claims 10 to 13, characterized in that the DNA fragment c) codes for an insect defensin.

**17.** An expression cassette according to Claim 16, characterized in that the DNA fragment c) codes for defensin A.

**18.** A plasmid vector comprising an expression cassette according to one of Claims 1 to 17.

**19.** A plasmid vector according to Claim 18, characterized in that it contains a fragment of the 2μ plasmid of yeast.

**20.** A plasmid vector according to Claim 18 or 19, characterized in that it contains, as a selectable gene, the URA3 gene from its promoter has been deleted.

**21.** A cell transformed by a plasmid vector according to one of Claims 18 to 20, or which has integrated in its genome an expression cassette according to one of Claims 10 to 17.

**22.** A yeast cell according to Claim 21.

**23.** A process for the preparation of a heterologous protein, characterized in that a cell according to Claim 21 or 22 is cultured and in that the said protein is recovered in the culture medium.

**24.** A process according to Claim 23, characterized in that the cell is a yeast cell.

**25.** A process according to Claim 23 or 24, characterized in that the said protein is a hirudin.

**26.** A process according to Claim 23 or 24, characterized in that the said protein is an insect defensin.

**Claims for the following Contracting State : ES**

**1.** Process for the preparation of an isolated DNA fragment which codes for a signal peptide useful for the secretion of a heterologous protein by a cell in which the heterologous protein is synthesized, characterized in that a DNA fragment is prepared which codes for a peptide whose amino acid sequence exhibits an at least 60% degree of homology with the amino acid sequence of formula (I) or (II)

(I)     Arg-Phe-Ser-Thr-Thr-Leu-Ala-Thr-Ala-Ala-Thr-Ala-Leu-Phe-Phe-Thr-Ala-Ser-Gln and

(II)     Arg-Phe-Ser-Thr-Thr-Leu-Ala-Thr-Ala-Ala-Thr-Ala-Leu-Phe-Phe-Thr-Ala-Ser-Gln-Val-Ser-Ala.

2. Process according to claim 1, characterized in that the DNA fragment codes for a peptide whose amino acid sequence exhibits an at least 80% degree of homology with the amino acid sequence (I) or (II) of formula

(I)     Arg-Phe-Ser-Thr-Thr-Leu-Ala-Thr-Ala-Ala-Thr-Ala-Leu-Phe-Phe-Thr-Ala-Ser-Gln and

(II)     Arg-Phe-Ser-Thr-Thr-Leu-Ala-Thr-Ala-Ala-Thr-Ala-Leu-Phe-Phe-Thr-Ala-Ser-Gln-Val-Ser-Ala.

3. Process according to either of Claims 1 and 2, characterized in that the DNA fragment codes for a peptide comprising the amino acid sequence (III)

(III)     $R_1$-$R_2$-$R_3$-Thr-Thr-$R_4$-Ala-Thr-Ala-Ala-Thr-Ala-Leu-
             1          5                    10
-Phe-Phe-Thr-Ala-$R_5$-$R_6$
     15          19

in which:
$R_1$ is an amino acid selected from Arg and Lys,
$R_2$ and $R_6$ are each an amino acid selected independently from Ala, Asn, Cys, Gln, Gly, His, Ile, Leu, Met, Phe, Pro, Ser, Thr, Trp, Tyr and Val,
$R_3$ and $R_5$ are each an amino acid selected independently from Asp, Gly, Asn, Pro and Ser, and
$R_4$ is an amino acid selected from Val, Leu, Ala, Cys, Phe, Ile and Met.

4. Process according to either of Claims 1 and 2, characterized in that the DNA fragment codes for a peptide comprising the amino acid sequence (IV)

$R_1$-$R_2$-$R_3$-Thr-Thr-$R_4$-Ala-Thr-Ala-Ala-Thr-Ala-Leu-Phe-Phe-Thr-Ala-$R_5$-$R_6$-$R_7$

in which:
$R_1$ is an amino acid selected from Arg and Lys,
$R_2$ and $R_6$ are each an amino acid selected independently from Ala, Asn, Cys, Gln, Gly, His, Ile, Leu, Met, Phe, Pro, Ser, Thr, Trp, Tyr and Val,
$R_3$ and $R_5$ are each an amino acid selected independently from Asp, Gly, Asn, Pro and Ser,
$R_4$ is an amino acid selected from Val, Leu, Ala, Cys, Phe, Ile and Met, and
$R_7$ is a proteolysis sequence.

23

5. Process according to Claim 4, in which $R_7$ is a proteolysis sequence $R_8$-$R_9$-$R_{10}$, in which:

$R_8$ is an amino acid selected from Ala, Val, Ser, Cys, Gly, Ile, Leu and Thr,

$R_9$ is an amino acid selected from Ala, Arg, Cys, Gln, Gly, His, Ile, Leu, Met, Phe, Pro, Ser, Thr, Trp, Tyr and Val and

$R_{10}$ is an amino acid selected from Ala, Cys, Gly, Leu, Pro, Gln, Ser and Thr.

6. Process according to either of Claims 1 and 2, characterized in that the DNA fragment codes for a peptide comprising an amino acid sequence selected from the amino acid sequences (V) and (VI)

(V)     Arg-Phe-Ser-Thr-Thr-Leu-Ala-Thr-Ala-Ala-Thr-Ala-

Leu-Phe-Phe-Thr-Ala-Ser-Gln;

(VI)     Arg-Phe-Ser-Thr-Thr-Val-Ala-Thr-Ala-Ala-Thr-Ala-

Leu-Phe-Phe-Thr-Ala-Ser-Gln.

7. Process according to one of Claims 1, 2, 4 and 5, characterized in that the DNA fragment codes for a peptide comprising an amino acid sequence selected from the amino acid sequences of formula (VII) and (VIII)

(VII)     Arg-Phe-Ser-Thr-Thr-Leu-Ala-Thr-Ala-Ala-Thr-Ala-

Leu-Phe-Phe-Thr-Ala-Ser-Gln-$R_7$,

in which $R_7$ is a proteolysis sequence:

(VIII)     Arg-Phe-Ser-Thr-Thr-Val-Ala-Thr-Ala-Ala-Thr-Ala-

Leu-Phe-Phe-Thr-Ala-Ser-Gln-$R_7$

in which $R_7$ is a proteolysis sequence.

8. Process according to Claim 7, characterized in that the DNA fragment codes for a peptide comprising an amino acid sequence selected from the amino acid sequences of formula (VII) and (VIII) in which $R_7$ is Val-Ser-Ala.

9. Application of a DNA fragment prepared according to one of Claims 1 to 8, characterized in that the DNA fragment codes for a signal peptide useful for the secretion of a heterologous protein by a yeast cell in which the heterologous protein is synthesized.

10. Process for the preparation of a cassette for the expression of a heterologous protein, characterized in that a cassette is prepared comprising at least:

a) a DNA fragment containing transcription and translation initiation signals,

b) a DNA fragment according to one of claims 1 to 8, and

c) a DNA fragment coding for the mature heterologous protein.

11. Process for the preparation of a cassette according to Claim 10, the said cassette comprising, in addition, a DNA fragment b') coding for a "pro" peptide fragment.

12. Process for the preparation of a cassette according to Claim 11, the said cassette comprising a DNA fragment b') coding for a "pro" peptide fragment having as its sequence

```
        Ala Pro Gly Leu Leu Phe Ile Asn Thr Thr
Ile Ala Ser Ile Ala Ala Lys Glu Glu Gly Val Ser Leu Asp
Lys Arg.
```

13. Process for the preparation of a cassette according to one of Claims 10 to 12, characterized in that the fragment a) contains a promoter which is functional in a yeast cell and an ATG translation initiation codon.

14. Process for the preparation of an expression cassette according to one of Claims 10 to 13, characterized in that the DNA fragment c) codes for a hirudin.

15. Process for the preparation of an expression cassette according to Claim 14, characterized in that the DNA fragment c) codes for the hirudin variant rHV2Lys47.

16. Process for the preparation of an expression cassette according to one of Claims 10 to 13, characterized in that the DNA fragment c) codes for an insect defensin.

17. Process for the preparation of an expression cassette according to Claim 16, characterized in that the DNA fragment c) codes for defensin A.

18. Process for the preparation of a plasmid vector, characterized in that an expression cassette according to one of Claims 10 to 17 is introduced.

19. Process for the preparation of a plasmid vector according to Claim 18, characterized in that the said plasmid contains a fragment of the 2$\mu$ plasmid of yeast.

20. Process for the preparation of a plasmid vector according to Claim 18 or 19, characterized in that the plasmid contains, as a selectable gene, the URA3 gene from its promoter has been deleted.

21. A cell transformed by a plasmid vector according to one of Claims 18 to 20, or which has integrated in its genome an expression cassette according to one of Claims 10 to 17.

22. A yeast cell according to Claim 21.

23. A process for the preparation of a heterologous protein, characterized in that a cell according to Claim 21 or 22 is cultured and in that the said protein is recovered in the culture medium.

24. A process according to Claim 23, characterized in that the cell is a yeast cell.

25. A process according to Claim 23 or 24, characterized in that the said protein is a hirudin.

26. A process according to Claim 23 or 24, characterized in that the said protein is an insect defensin.

**Patentansprüche**
**Patentansprüche für folgende Vertragsstaaten : AT, BE, CH, DE, DK, FR, GB, IT, LI, LU, NL, SE**

1. Isoliertes DNA-Fragment, das für ein Signal-Peptid codiert, das geeignet ist für die Sekretion eines heterologen Proteins durch eine Zelle, in der das heterologe Protein synthetisiert wird, dessen Aminosäuresequenz einen Grad der Homologie von mindestens 60 % mit der Aminosäuresequenz der Formel (I) oder (II) aufweist

(I)     Arg-Phe-Ser-Thr-Thr-Leu-Ala-Thr-Ala-Ala-Thr-Ala-Leu-Phe-Phe-Thr-Ala-Ser-Gln und

(II)    Arg-Phe-Ser-Thr-Thr-Leu-Ala-Thr-Ala-Ala-Thr-Ala-Leu-Phe-Phe-Thr-Ala-Ser-Gln-Val-Ser-Ala.

2.  DNA-Fragment nach Anspruch 1, das für ein Peptid codiert, dessen Aminosäuresequenz einen Grad der Homologie von mindestens 80 % mit der Aminosäuresequenz der Formel (I) oder (II) aufweist

(I)     Arg-Phe-Ser-Thr-Thr-Leu-Ala-Thr-Ala-Ala-Thr-Ala-Leu-Phe-Phe-Thr-Ala-Ser-Gln und

(II)    Arg-Phe-Ser-Thr-Thr-Leu-Ala-Thr-Ala-Ala-Thr-Ala-Leu-Phe-Phe-Thr-Ala-Ser-Gln-Val-Ser-Ala.

3.  DNA-Fragment nach Anspruch 1 oder 2, das für ein Peptid codiert, das die Aminosäuresequenz (III) aufweist

(III) $R_1$-$R_2$-$R_3$-Thr-Thr-$R_4$-Ala-Thr-Ala-Ala-Thr-Ala-Leu-Phe-
            1                 5                          10

Phe-Thr-Ala-$R_5$-$R_6$
     15              19

in der:
$R_1$           steht für eine Aminosäure, ausgewählt aus Arg und Lys,
$R_2$ und $R_6$   jeweils stehen für eine Aminosäure, unabhängig ausgewählt aus Ala, Asn, Cys, Gln, Gly, His, Ile, Leu, Met, Phe, Pro, Ser, Thr, Trp, Tyr und Val
$R_3$ und $R_5$   jeweils stehen für eine Aminosäure, unabhängig ausgewählt aus Asp, Gly, Asn, Pro und Ser und
$R_4$           steht für eine Aminosäure, ausgewählt aus Val, Leu, Ala, Cys, Phe, Ile und Met.

4.  DNA-Fragment nach Anspruch 1 oder 2, das für ein Peptid codiert, das die Aminosäuresequenz (IV) aufweist

(IV)    $R_1$-$R_2$-$R_3$-Thr-Thr-$R_4$-Ala-Thr-Ala-Ala-Thr-Ala-Leu-Phe-Phe-Thr-Ala-$R_5$-$R_6$-$R_7$

in der:
$R_1$           steht für eine Aminosäure, ausgewählt aus Arg und Lys,
$R_2$ und $R_6$   jeweils stehen für eine Aminosäure, unabhängig ausgewählt aus Ala, Asn, Cys, Gln, Gly, His, Ile, Leu, Met, Phe, Pro, Ser, Thr, Trp, Tyr und Val
$R_3$ und $R_5$   stehen jeweils für eine Aminosäurese, unabhängig ausgewählt aus Asp, Gly, Asn, Pro und Ser,
$R_4$           steht für eine Aminosäure, ausgewählt aus Val, Leu, Ala, Cys, Phe, Ile, et Met, und
$R_7$           steht für eine Proteolyse-Sequenz.

26

5. DNA-Fragment nach Anspruch 4, in dem R$_7$ eine Proteolyse-Sequenz R$_8$-R$_9$-R$_{10}$ ist, in der:

R$_8$ steht für eine Aminosäure, ausgewählt aus Ala, Val, Ser, Cys, Gly, Ile, Leu und Thr,

R$_9$ steht für eine Aminosäure, ausgewählt aus Ala, Arg, Cys, Gln, Gly, His, Ile, Leu, Met, Phe, Pro, Ser, Thr, Trp, Tyr und Val und

R$_{10}$ steht für eine Aminosäure, ausgewählt aus Ala, Cys, Gly, Leu, Pro, Gln, Ser und Thr.

6. DNA-Fragment nach Anspruch 1 oder 2, das für ein Peptid codiert, das eine Aminosäuresequenz enthält, die ausgewählt wird aus den Aminosäuresequenzen (V) und (VI)

(V)    Arg-Phe-Ser-Thr-Thr-Leu-Ala-Thr-Ala-Ala-Thr-Ala-Leu-Phe-Phe-Thr-Ala-Ser-Gln;

(VI)    Arg-Phe-Ser-Thr-Thr-Val-Ala-Thr-Ala-Ala-Thr-Ala-Leu-Phe-Phe-Thr-Ala-Ser-Gln.

7. DNA-Fragment nach einem der Ansprüche 1, 2, 4 und 5, das für ein Peptid codiert, das eine Aminosäuresequenz aufweist, die ausgewählt wird aus den Aminosäuresequenzen der Formel (VII) und (VIII)

(VII) Arg-Phe-Ser-Thr-Thr-Leu-Ala-Thr-Ala-Ala-Thr-Ala-Leu-Phe-Phe-Thr-Ala-Ser-Gln-R$_7$

in der R$_7$ eine Proteolyse-Sequenz darstellt;

(VIII) Arg-Phe-Ser-Thr-Thr-Val-Ala-Thr-Ala-Ala-Thr-Ala-Leu-Phe-Phe-Thr-Ala-Ser-Gln-R$_7$

in der R$_7$ eine Proteolyse-Sequenz darstellt.

8. DNA-Fragment nach Anspruch 7, das für ein Peptid codiert, das eine Aminosäuresequenz aufweist, die ausgewählt wird aus den Aminosäuresequenzen der Formel (VII) und (VIII), in denen R$_7$ für Val-Ser-Ala steht.

9. Verwendung eines DNA-Fragments nach einem der Ansprüche 1 bis 8, dadurch gekennzeichnet, daß das DNA-Fragment für ein Signal-Peptid codiert, das für die Sekretion eines heterologen Proteins durch eine Hefezelle geeignet ist, in der das heterologe Protein synthetisiert wird.

10. Kassette für die Expression eines heterologen Proteins, die umfaßt:

a) ein DNA-Fragment, das Transkriptions- und Translations-Initiierungssignale aufweist,

b) ein DNA-Fragment nach einem der Ansprüche 1 bis 8 und

c) ein DNA-Fragment, das für das reife heterologe Protein codiert.

11. Kassette nach Anspruch 10, die außerdem ein DNA-Fragment (b') aufweist, das für ein Peptidfragment "pro" codiert.

12. Kassette nach Anspruch 11, die ein DNA-Fragment (b') aufweist, das für ein Peptidfragment "pro" codiert, das die Sequenz hat

Ala Pro Gly Leu Leu Phe Ile Asn Thr Thr Ile Ala Ser Ile Ala Ala Lys Glu Glu Gly Val Ser Leu Asp Lys Arg.

13. Kassette nach einem der Ansprüche 10 bis 12, dadurch gekennzeichnet, daß das Fragment (a) einen funktionellen Promotor in einer Hefezelle und einen Translations-Initiierungscodon ATG aufweist.

14. Expressions-Kassette nach einem der Ansprüche 10 bis 13, dadurch gekennzeichnet, daß das DNA-Fragment (c) für ein Hirudin codiert.

15. Expressions-Kassette nach Anspruch 14, dadurch gekennzeichnet, daß das DNA-Fragment (c) für die Hirudin-Variante rHV2Lys47 codiert.

16. Expressions-Kassette nach einem der Ansprüche 10 bis 13, dadurch gekennzeichnet, daß das DNA-Fragment (c) für ein Insekten-Defensin codiert.

17. Expressions-Kassette nach Anspruch 16, dadurch gekennzeichnet, daß das DNA-Fragment (c) für Defensin A codiert.

18. Plasmid-Vektor, der eine Expressions-Kassette nach einem der Ansprüche 10 bis 17 enthält.

19. Plasmid-Vektor nach Anspruch 18, dadurch gekennzeichnet, daß er ein Fragment des Hefe-Plasmids 2 μ enthält.

20. Plasmid-Vektor nach Anspruch 18 oder 19, dadurch gekennzeichnet, daß er als Selektions-Gen das Gen URA3 enthält, aus dem sein Promotor deletiert worden ist.

21. Zelle, die durch einen Plasmid-Vektor nach einem der Ansprüche 18 bis 20 transformiert worden ist oder in deren Genom eine Expressions-Kassette nach einem der Ansprüche 10 bis 17 integriert worden ist.

22. Hefezelle nach Anspruch 21.

23. Verfahren zur Herstellung eines heterologen Proteins, dadurch gekennzeichnet, daß man eine Zelle nach Anspruch 21 oder 22 kultiviert und daß man das genannte Protein aus dem Kulturmedium gewinnt (abtrennt).

24. Verfahren nach Anspruch 23, dadurch gekennzeichnet, daß es sich bei der Zelle um eine Hefezelle handelt.

25. Verfahren nach Anspruch 23 oder 24, dadurch gekennzeichnet, daß das genannte Protein ein Hirudin ist.

26. Verfahren nach Anspruch 23 oder 24, dadurch gekennzeichnet, daß das genannte Protein ein Insekten-Defensin ist.

**Patentansprüche für folgenden Vertragsstaat : ES**

1. Verfahren zur Herstellung eines isolierten DNA-Fragments, das für ein Signal-Peptid codiert, das geeignet ist für die Sekretion eines heterologen Proteins durch eine Zelle, in der das heterologe Protein synthetisiert wird, dadurch gekennzeichnet, daß man ein DNA-Fragment herstellt, das für ein Peptid codiert, dessen Aminosäuresequenz einen Grad der Homologie von mindestens 60 % mit der Aminosäuresequenz der Formel (I) oder (II) aufweist

(I)    Arg-Phe-Ser-Thr-Thr-Leu-Ala-Thr-Ala-Ala-Thr-Ala-Leu-Phe-Phe-Thr-Ala-Ser-Gln **und**

(II)    Arg-Phe-Ser-Thr-Thr-Leu-Ala-Thr-Ala-Ala-Thr-Ala-Leu-Phe-Phe-Thr-Ala-Ser-Gln-Val-Ser-Ala.

2. Verfahren nach Anspruch 1, dadurch gekennzeichnet, daß das DNA-Fragment für ein Peptid codiert, dessen Aminosäuresequenz einen Grad der Homologie von mindestens 80 % mit der Aminosäuresequenz der Formel (I) oder (II) aufweist

(I)    Arg-Phe-Ser-Thr-Thr-Leu-Ala-Thr-Ala-Ala-Thr-Ala-Leu-Phe-Phe-Thr-Ala-Ser-Gln **und**

(II)    Arg-Phe-Ser-Thr-Thr-Leu-Ala-Thr-Ala-Ala-Thr-Ala-Leu-Phe-Phe-Thr-Ala-Ser-Gln-Val-Ser-Ala.

3. Verfahren nach einem der Ansprüche 1 oder 2, dadurch gekennzeichnet, daß das DNA-Fragment für eine Peptid codiert, das die Aminosäuresequenz (III) aufweist

(III) $R_1$-$R_2$-$R_3$-Thr-Thr-$R_4$-Ala-Thr-Ala-Ala-Thr-Ala-Leu-Phe-
                1            5                    10

Phe-Thr-Ala-$R_5$-$R_6$
        15            19

in der:
|  |  |
|---|---|
| $R_1$ | steht für eine Aminosäure, ausgewählt aus Arg und Lys, |
| $R_2$ und $R_6$ | jeweils stehen für eine Aminosäure, unabhängig ausgewählt aus Ala, Asn, Cys, Gln, Gly, His, Ile, Leu, Met, Phe, Pro, Ser, Thr, Trp, Tyr und Val |
| $R_3$ und $R_5$ | jeweils stehen für eine Aminosäure, unabhängig ausgewählt aus Asp, Gly, Asn, Pro und Ser und |
| $R_4$ | steht für eine Aminosäure, ausgewählt aus Val, Leu, Ala, Cys, Phe, Ile und Met. |

4. Verfahren nach einem der Ansprüche 1 oder 2, dadurch gekennzeichnet, daß das DNA-Fragment codiert für ein Peptid, das die Aminosäuresequenz (IV) aufweist

(IV)    $R_1$-$R_2$-$R_3$-Thr-Thr-$R_4$-Ala-Thr-Ala-Ala-Thr-Ala-Leu-Phe-Phe-Thr-Ala-$R_5$-$R_6$-$R_7$

in der:
|  |  |
|---|---|
| $R_1$ | steht für eine Aminosäure, ausgewählt aus Arg und Lys, |
| $R_2$ und $R_6$ | jeweils stehen für eine Aminosäure, unabhängig ausgewählt aus Ala, Asn, Cys, Gln, Gly, His, Ile, Leu, Met, Phe, Pro, Ser, Thr, Trp, Tyr und Val |
| $R_3$ und $R_5$ | stehen jeweils für eine Aminosäurese, unabhängig ausgewählt aus Asp, Gly, Asn, Pro und Ser |
| $R_4$ | steht für eine Aminosäure, ausgewählt aus Val, Leu, Ala, Cys, Phe, Ile und Met, und |
| $R_7$ | steht für eine Proteolyse-Sequenz. |

29

5. Verfahren nach Anspruch 4, in dem R$_7$ eine Proteolyse-Sequenz R$_8$-R$_9$-R$_{10}$ ist, in der:
   - R$_8$ steht für eine Aminosäure, ausgewählt aus Ala, Val, Ser, Cys, Gly, Ile, Leu, und Thr,
   - R$_9$ steht für eine Aminosäure, ausgewählt aus Ala, Arg, Cys, Gln, Gly, His, Ile, Leu, Met, Phe, Pro, Ser, Thr, Trp, Tyr und Val und
   - R$_{10}$ steht für eine Aminosäure, ausgewählt aus Ala, Cys, Gly, Leu, Pro, Gln, Ser und Thr.

6. Verfahren nach einem der Ansprüche 1 oder 2, dadurch gekennzeichnet, daß das DNA-Fragment codiert für ein Peptid, das eine Aminosäuresequenz enthält, die ausgewählt wird aus den Aminosäuresequenzen (V) und (VI)

(V) Arg-Phe-Ser-Thr-Thr-Leu-Ala-Thr-Ala-Ala-Thr-Ala-Leu-Phe-Phe-Thr-Ala-Ser-Gln;

(VI) Arg-Phe-Ser-Thr-Thr-Val-Ala-Thr-Ala-Ala-Thr-Ala-Leu-Phe-Phe-Thr-Ala-Ser-Gln.

7. Verfahren nach einem der Ansprüche 1, 2, 4 oder 5, dadurch gekennzeichnet, daß das DNA-Fragment codiert für ein Peptid, das eine Aminosäuresequenz aufweist, die ausgewählt wird aus den Aminosäuresequenzen der Formel (VII) und (VIII)

(VII) Arg-Phe-Ser-Thr-Thr-Leu-Ala-Thr-Ala-Ala-Thr-Ala-Leu-Phe-Phe-Thr-Ala-Ser-Gln-R$_7$

in der R$_7$ eine Proteolyse-Sequenz darstellt;

(VIII) Arg-Phe-Ser-Thr-Thr-Val-Ala-Thr-Ala-Ala-Thr-Ala-Leu-Phe-Phe-Thr-Ala-Ser-Gln-R$_7$

in der R$_7$ eine Proteolyse-Sequenz darstellt.

8. Verfahren nach Anspruch 7, dadurch gekennzeichnet, daß das DNA-Fragment codiert für ein Peptid, das eine Aminosäuresequenz aufweist, die ausgewählt wird aus den Aminosäuresequenzen der Formel (VII) und (VIII), in denen R$_7$ für Val-Ser-Ala steht.

9. Verwendung eines DNA-Fragments nach einem der Ansprüche 1 bis 8, dadurch gekennzeichnet, daß das DNA-Fragment für ein Signal-Peptid codiert, das für die Sekretion eines heterologen Proteins durch eine Hefezelle geeignet ist, in der das heterologe Protein synthetisiert wird.

10. Verfahren zur Herstellung einer Kassette für die Expression eines heterologen Proteins, dadurch gekennzeichnet, daß man eine Kassette herstellt, die umfaßt mindestens:
    a) ein DNA-Fragment, das Transkriptions- und Translations-Initiierungssignale aufweist,
    b) ein DNA-Fragment nach einem der Ansprüche 1 bis 8 und
    c) ein DNA-Fragment, das für das reife heterologe Protein codiert.

11. Verfahren zur Herstellung einer Kassette nach Anspruch 10, wobei die Kassette außerdem ein DNA-Fragment (b') umfaßt, das für ein Peptidfragment "pro" codiert.

12. Verfahren zur Herstellung einer Kassette nach Anspruch 11, wobei die Kassette umfaßt ein DNA-Fragment (b'), das für ein Peptidfragment "pro" codiert, das die Sequenz hat

```
            Ala Pro Gly Leu Leu Phe
Ile Asn Thr·Thr Ile Ala Ser Ile Ala Ala Lys Glu Glu Gly Val Ser Leu Asp Lys

    Arg.
```

**13.** Verfahren zur Herstellung einer Kassette nach einem der Ansprüche 10 bis 12, dadurch gekennzeichnet, daß das Fragment (a) einen funktionellen Promotor in einer Hefezelle und einen Translationsinitiierungscodon ATG aufweist.

**14.** Verfahren zur Herstellung einer Expressions-Kassette nach einem der Ansprüche 10 bis 13, dadurch gekennzeichnet, daß das DNA-Fragment (c) für ein Hirudin codiert.

**15.** Verfahren zur Herstellung einer Expressions-Kassette nach Anspruch 14, dadurch gekennzeichnet, daß das DNA-Fragment (c) für die Hirudin-Variante rHV2Lys47 codiert.

**16.** Verfahren zur Herstellung einer Expressions-Kassette nach einem der Ansprüche 10 bis 13, dadurch gekennzeichnet, daß das DNA-Fragment (c) für ein Insekten-Defensin codiert.

**17.** Verfahren zur Herstellung einer Expressions-Kassette nach Anspruch 16, dadurch gekennzeichnet, daß das DNA-Fragment (c) für das Defensin A codiert.

**18.** Verfahren zur Herstellung eines Plasmid-Vektors, dadurch gekennzeichnet, daß man eine Expressions-Kassette nach einem der Ansprüche 10 bis 17 einführt.

**19.** Verfahren zur Herstellung eines Plasmid-Vektors nach Anspruch 18, dadurch gekennzeichnet, daß das genannte Plasmid ein Fragment des Hefe-Plasmids 2 μ aufweist.

**20.** Verfahren zur Herstellung eines Plasmid-Vektors nach Anspruch 18 oder 19, dadurch gekennzeichnet, daß das Plasmid als Selektions-Gen das Gen URA3 aufweist, aus dem sein Promotor deletiert worden ist.

**21.** Zelle, die durch einen Plasmid-Vektor nach einem der Ansprüche 18 bis 20 transformiert worden ist oder in deren Genom eine Expression-Kassette nach einem der Ansprüche 10 bis 17 integriert worden ist.

**22.** Hefezelle nach Anspruch 21.

**23.** Verfahren zur Herstellung eines heterologen Proteins, dadurch gekennzeichnet, daß man eine Zelle nach Anspruch 21 oder 22 kultiviert und daß man das genannte Protein aus dem Kulturmedium gewinnt (abtrennt).

**24.** Verfahren nach Anspruch 23, dadurch gekennzeichnet, daß es sich bei der Zelle um eine Hefezelle handelt.

**25.** Verfahren nach Anspruch 23 oder 24, dadurch gekennzeichnet, daß das genannte Protein ein Hirudin ist.

**26.** Verfahren nach Anspruch 23 oder 24, dadurch gekennzeichnet, daß das genannte Protein ein Insekten-Defensin ist.

# FIG.1

FIG. 2

M13TG3839

HindIII

URA3-d

Promoteur

Prepro

MFalpha1

rHV2 Lys47

HindIII

M13TG3841

SphI

Promoteur

Prepro

MFalpha1

rHV2 Lys47

SalI

FIG. 3

M13TG 3845

FIG. 4

M13TG131

pTG 3828

FIG. 5

Sph I

Sall

| Promoteur MFα1 | Peptide I | Pro-d MFα1 | rHV2 Lys 47 |

Hind III

Hind III

| URA3-d | | terminateur PGK | pBR322 | 2 μ |

pTG 3864

EP 0 423 302 B1